(19) Europäisches Patentamt
European Patent Office
Office européen des brevets

(11) Numéro de publication: **0 425 345 B1**

(12) # FASCICULE DE BREVET EUROPEEN

(45) Date de publication de fascicule du brevet: **14.09.94**

(51) Int. Cl.⁵: **A61K 7/13**, C07D 209/08

(21) Numéro de dépôt: **90402927.9**

(22) Date de dépôt: **18.10.90**

Le dossier contient des informations techniques présentées postérieurement au dépôt de la demande et ne figurant pas dans le présent fascicule.

(54) **Procédés de teinture des fibres kératiniques avec des amino indoles, compositions et dispositifs de mise en oeuvre.**

(30) Priorité: **20.10.89 FR 8913788**
**16.03.90 FR 9003436**

(43) Date de publication de la demande:
**02.05.91 Bulletin 91/18**

(45) Mention de la délivrance du brevet:
**14.09.94 Bulletin 94/37**

(84) Etats contractants désignés:
**AT BE CH DE DK ES FR GB GR IT LI NL SE**

(56) Documents cités:
**EP-A- 0 271 186**
**EP-A- 0 348 280**
**FR-A- 2 626 173**
**US-A- 4 013 404**

(73) Titulaire: **L'OREAL**
**14, Rue Royale**
**F-75008 Paris (FR)**

(72) Inventeur: **Lang, Gérard**
**44, avenue Lacour**
**F-95210 Saint-Gratien (FR)**
Inventeur: **Junino, Alex**
**16, rue Docteur Bergonié**
**F-93180 Livry-Gargan (FR)**
Inventeur: **Cotteret, Jean**
**15 Allée des Meuniers**
**F-78480 Verneuil Sur Seine (FR)**

(74) Mandataire: **Casalonga, Axel et al**
**BUREAU D.A. CASALONGA - JOSSE**
**Morassistrasse 8**
**D-80469 München (DE)**

EP 0 425 345 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à de nouveaux procédés de teinture des fibres kératiniques et en particulier des fibres kératiniques humaines, telles que les cheveux, en mettant en oeuvre un amino indole ainsi qu'aux compositions tinctoriales et aux dispositifs permettant de mettre en oeuvre ces procédés.

Les colorants de la famille des indoles et en particulier le 5,6-dihydroxyindole ainsi que ses dérivés sont bien connus pour leur utilisation en teinture des fibres kératiniques et notamment des cheveux humains.

Les brevets français FR-A-1 133 594, 1 166 172 et 2 390 158 proposent des procédés de teinture à l'aide du 5,6-dihydroxyindole, en mettant en oeuvre des cations métalliques jouant le rôle de promoteur de la mélanogénèse.

Le 5,6-dihydroxyindole conduit en particulier à des teintres noires ou plus ou moins grises.

Dans sa demande de brevet FR-A-2 626 173, la demanderesse emploie des dérivés du 5,6-dihydroxyindole en association avec des dérivés quinoniques dans un procédé de teinture par oxydation, dans lequel la couleur est révélée au moyen de systèmes oxydants de préférence inorganiques.

On a également décrit dans les FR-A-2 593 061, 2 593 062 et 2 594 331, différents procédés de teinture, mettant en oeuvre des dérivés d'indole, en association avec les systèmes oxydants, tels que des anions minéraux, comme par exemple l'iodure ou des anions métalliques, tels que le permanganate ou le bichromate.

La demande de brevet EP-A-0 271 186 décrit des procédés de teinture des cheveux mettant en oeuvre des compositions tinctoriales contenant une monohydroxyindole à titre de colorant et un agent oxydant, essentiellement le périodate de sodium.

Le brevet US 4 013 404 décrit un procédé de teinture en une seule étape mettant en oeuvre une composition tinctoriale contenant un composé indolique et un agent oxydant.

Les inventeurs viennent de découvrir qu'une classe particulière d'indoles substitués sur le noyau aromatique par une fonction amine permettait, lorsque la coloration est développée par des agents oxydants, d'obtenir des nuances particulièrement intéressantes et puissantes.

L'invention a donc pour objet un procédé de teinture des fibres kératiniques et en particulier des fibres kératiniques humaines, en mettant en oeuvre des amino indoles en association avec un système oxydant.

Un autre objet de l'invention est constitué par les compositions tinctoriales mises en oeuvre au cours de ce procédé, ainsi qu'au dispositif destiné à être utilisé dans la mise en oeuvre de ce procédé.

D'autres objets de l'invention apparaîtront à la lecture de la description et des exemples qui suivent.

Le procédé de teinture des fibres kératiniques et en particulier des fibres kératiniques humaines, telles que les cheveux, conforme à l'invention, est caractérisé par le fait que l'on applique sur ces fibres une composition (A) contenant, dans un milieu approprié pour la teinture, au moins un amino indole répondant à la formule :

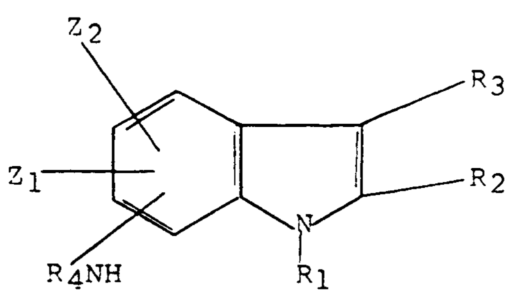

(I)

dans laquelle :

$R_1$ et $R_3$, indépendamment l'un de l'autre, représentent un atome d'hydrogène ou un groupement alkyle en $C_1$-$C_4$;

$R_2$ désigne hydrogène ou un groupement alkyle en $C_1$-$C_4$, COOR′, R′ étant un atome d'hydrogène ou un groupement alkyle en $C_1$-$C_4$;

$R_4$ représente un atome d'hydrogène ou un groupement alkyle en $C_1$-$C_4$, hydroxyalkyle en $C_1$-$C_4$, polyhydroxyalkyle en $C_2$-$C_4$;

$Z_1$ représente un atome d'hydrogène ou d'halogène, un groupement alkyle en $C_1$-$C_4$, ou OR;

R étant un atome d'hydrogène ou un groupement alkyle en $C_1$-$C_4$;

$Z_2$ représente un atome d'hydrogène ou un groupement alkyle en $C_1$-$C_4$;

et ses sels,

la couleur étant révélée à l'aide d'un système oxydant constitué par :

(i) des ions iodure et du peroxyde d'hydrogène, la composition (A) contenant dans ce cas en plus, soit (a) des ions iodure, soit (b) du peroxyde d'hydrogène et l'application de la composition (A) étant précédée ou suivie par l'application d'une composition (B) qui contient, dans un milieu approprié pour la teinture, soit :

(a) du peroxyde d'hydrogène à un pH compris entre 2 et 12 et de préférence entre 2 et 7, lorsque la composition (A) contient des ions iodure, soit

(b) des ions iodure à un pH compris entre 3 et 11, lorsque la composition (A) contient du peroxyde d'hydrogène;

(ii) des nitrites, l'application de la composition (A) étant suivie par l'application d'une composition aqueuse (B) présentant un pH acide, la composition (A) ou la composition (B) contenant au moins un nitrite;

(iii) des oxydants choisis parmi l'acide périodique et ses sels hydrosolubles, l'hypochlorite de sodium, le ferricyanure de potassium, l'oxyde d'argent, le réactif de Fenton, l'oxyde de plomb (IV), le sulfate de césium, le persulfate d'ammonium, ces oxydants étant présents dans la composition (A) ou appliqués simultanément ou séquentiellement de façon séparée, au moyen d'une composition (B) les contenant dans un milieu approprié pour la teinture;

(iv) des anions d'un métal choisi parmi les permanganates ou bichromates, ces oxydants étant appliqués au moyen d'une composition (B) aqueuse à un pH de 2 à 10, avant l'application de la composition (A);

(v) des sels métalliques des groupes III à VIII du tableau périodique, ces sels métalliques étant appliqués dans une étape séparée au moyen d'une composition (B) contenant ces sels dans un milieu approprié pour la teinture;

(vi) des sels de terres rares, ces sels de terres rares étant appliqués au moyen d'une composition (B) les contenant dans un milieu approprié pour la teinture, la composition (B) étant appliquée préalablement ou postérieurement à l'application de la composition (A).

Conformément à l'invention, l'application des compositions (A) et (B) est de préférence séparée par un rinçage.

Parmi les dérivés d'amino indole répondant à la formule (I), on peut citer le 4-amino indole, le 5-amino indole, le 6-amino indole, le 7-amino indole, le 5-amino 6-méthoxy 2,3-diméthyl indole, le 6-amino 5-méthoxy 2,3-diméthyl indole, le 5-amino 6-hydroxy 2,3-diméthyl indole, le 5-hydroxy 6-amino 2,3-diméthyl indole, le 6-N-$\beta$-hydroxyéthylamino indole, le 6-N-$\beta$-hydroxyéthylamino 1-méthyl indole, le 6-méthylamino indole, le (5 ou 6)-amino N-méthyl indole, le 2-carboxy 6-amino indole, la 4-amino 2,3-diméthyl indole, le 6-amino 2,3-diméthyl indole, le 7-amino 2,3-diméthyl indole, le 6-amino 3-éthyl 2-méthyl indole, le 6-amino 3-méthyl indole, le 6-amino 2-méthyl indole, le 6-amino 2-éthoxycarbonyl indole, le 7-amino 3-éthyl 2-méthyl indole, le 6-N-($\beta$,$\gamma$-dihydroxypropyl)aminoindole, le 2,3,4,5-tétraméthyl 6-amino indole, le 2,3-diméthyl 5-chloro 6-amino indole, le 2,3-diméthyl 5-éthyl 6-amino indole, le 2,3,4-triméthyl 6-amino indole, le 2-méthyl 5-hydroxy 6-amino indole, le 4-méthylamino indole, le 4-amino 1-méthyl indole, le 2,3-diméthyl 6-amino indole, le 2,3,7-triméthyl 6-amino indole, le 2,3,5-triméthyl 6-amino indole, et leurs sels.

Les composés nouveaux entrant dans la formule (I) répondent à la formule (IA) :

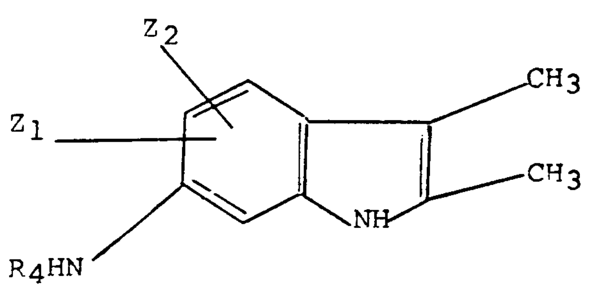

(IA)

dans laquelle :

$R_4$ désigne hydrogène, alkyle en $C_1$-$C_4$, hydroxyalkyle en $C_1$-$C_4$, polyhydroxyalkyle en $C_2$-$C_4$;

$Z_1$ désigne hydrogène, alkyle ou halogène;

$Z_2$ désigne hydrogène, alkyle en $C_1$-$C_4$, sous réserve qu'au moins un des radicaux $Z_1$ ou $Z_2$ soit différent d'hydrogène, à l'exclusion du 2,3,5-triméthyl 6-amino indole.

Parmi les composés nouveaux, on peut citer en particulier le 2,3,7-triméthyl 6-amino indole, le 2,3,4,5-tétraméthyl 6-amino indole, le 2,3-diméthyl 5-éthyl 6-amino indole, le 2,3-diméthyl 5-chloro 6-amino indole,

le 2,3,4-triméthyl 6-amino indole.

Les composés de formule (IA) dans lesquels $R_4$ désigne hydrogène, sont préparés selon le schéma réactionnel suivant :

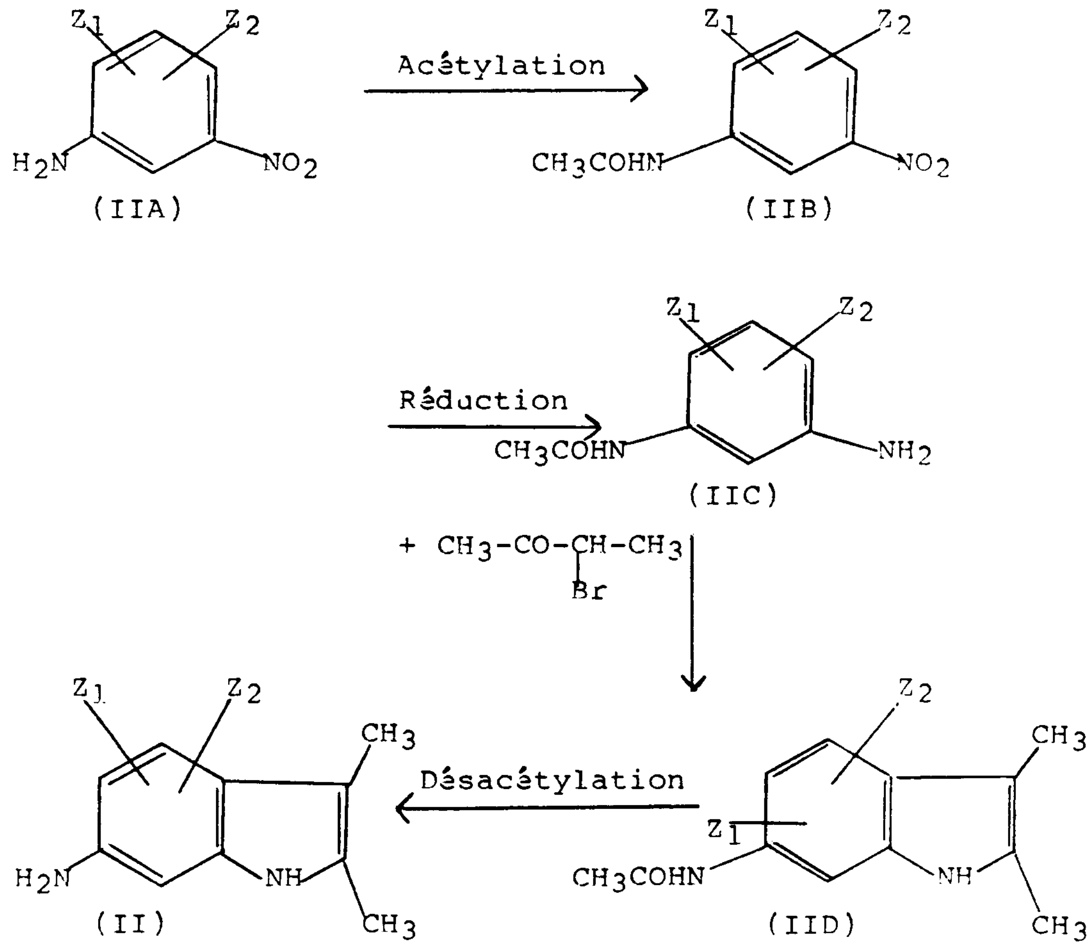


1ère étape :

Elle consiste en une acétylation de la méta-nitroaniline mono- ou disubstituée de formule (IIA). Il s'agit d'une acétylation classique à l'anhydride acétique dans un solvant, comme l'acétate d'éthyle, à reflux du solvant.

2ème étape :

Le composé acétylé (IIB) est réduit selon les procédés classiques. Cette réduction s'effectue soit par le fer acétique dans l'eau à une température variant entre 50° et 95°C, soit par transfert d'hydrogène en utilisant comme catalyseur le Pd/C en présence de cyclohexène, dans un solvant à une température comprise entre la température ambiante et la température de reflux du solvant, (comme solvant, on peut citer les alcools inférieurs en $C_1$-$C_4$) ou encore par hydrogénation catalytique en utilisant le Pd/C ou le nickel de Raney comme catalyseur.

3ème étape :

Sur le composé (IIC), on effectue la condensation de la 3-bromo 2-butanone dans le diméthylformamide à une température comprise entre la température ambiante et 120°C.

4ème étape :

Le composé (IID) est désacétylé, en présence d'acide chlorhydrique concentré à chaud. On obtient ainsi les composés (II).

Le composé (IA) dans lequel $R_4$ est différent d'hydrogène, est obtenu à partir du composé (II) ($R_4 = H$) par les méthodes de substitution des amines aromatiques, selon le schéma réactionnel :

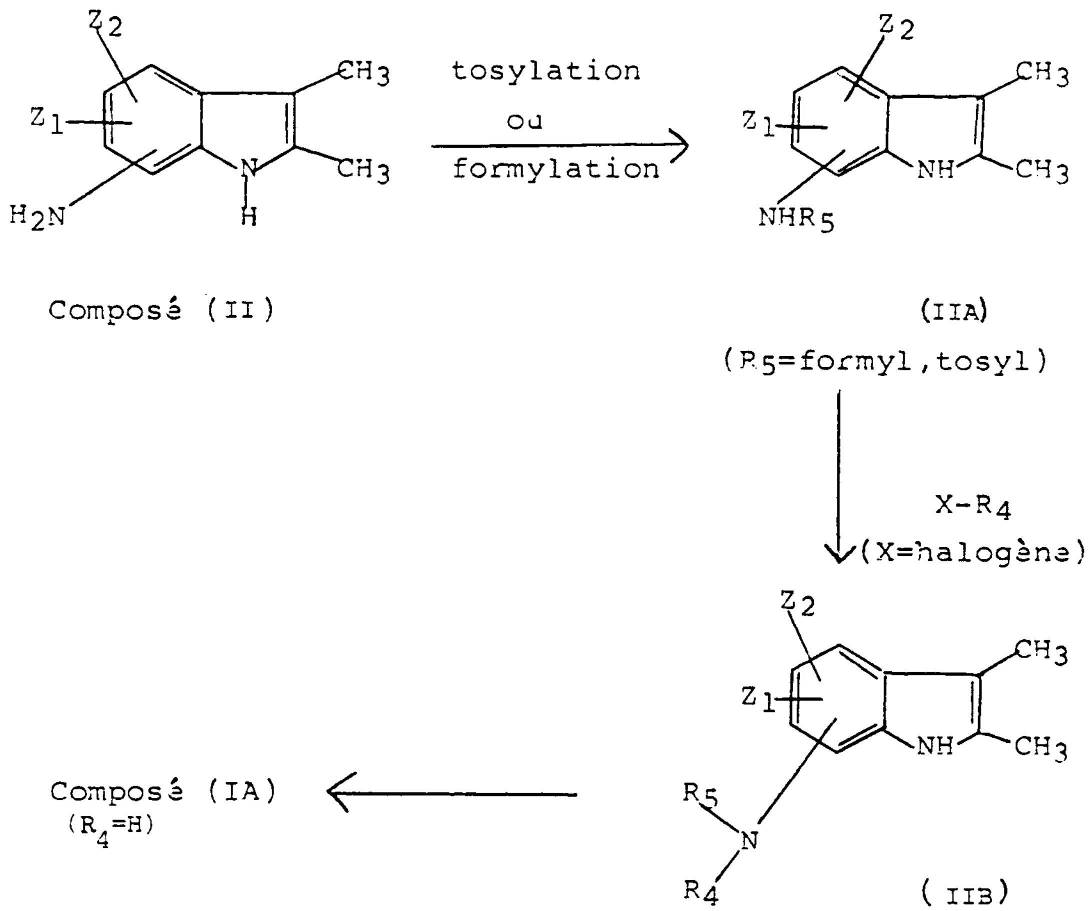


Par formylation ou tosylation, on obtient le composé (IIA). Le composé (IIA) est alkylé dans un deuxième temps par l'halogénure d'alkyle X-$R_4$. Lorsque l'halogénure d'alkyle est utilisé en excès, un second groupement $R_4$ est introduit. On obtient le produit (IA) par déformylation ou détosylation du composé (IIB).

Parmi les méthodes d'hydroxylation, on peut citer l'action du chloroformiate de $\beta$-chloréthyle sur le composé (II) qui permet d'obtenir dans un premier temps, le carbamate de $\beta$-chloréthyle correspondant qui soumis dans un deuxième temps à l'action d'une base minérale forte, permet d'obtenir le composé (IA) pour lequel le radical $R_4$ est un radical $\beta$-hydroxyéthyle.

Selon une première variante de l'invention, on applique sur les matières kératiniques une composition (A) contenant, dans un milieu approprié pour la teinture, au moins un colorant de formule (I) en association avec des ions iodure, l'application de la composition (A) étant précédée ou suivie par l'application d'une composition (B) qui contient, dans un milieu approprié pour la teniture, du peroxyde d'hydrogène.

Ce procédé peut également être mis en oeuvre en appliquant sur les fibres kératiniques au moins une composition (A) contenant, dans un milieu approprié pour la teinture, le colorant de formule (I), en association avec du peroxyde d'hydrogène, ayant un pH compris entre 2 et 7 et de préférence entre 3,5 et 7, l'application de la composition (A) étant précédée ou suivie par l'application d'une composition (B) qui contient dans un milieu approprié pour la teinture, des ions iodure.

L'ion iodure dans cette variante du procédé conforme à l'invention, est choisi de préférence parmi les iodures de métaux alcalins, alcalino-terreux ou d'ammonium et plus particulièrement est constitué par l'iodure de potassium.

Les ions iodure sont présents dans les compositions (A) ou (B) dans des proportions comprises généralement entre 0,007 et 4% en poids exprimé en ions $I^-$ et de préférence entre 0,08 et 1,5% en poids par rapport au poids total de la composition (A) ou (B).

Selon une seconde variante de l'invention, on peut mettre en oeuvre le procédé en utilisant comme agent oxydant pour révéler la coloration, un nitrite. Les nitrites plus particulièrement utilisables, conformément à l'invention, sont :

- des nitrites de métaux alcalins, alcalinoterreux ou d'ammonium ou de tout autre cation cosmétiquement acceptable, lorsqu'il est utilisé pour la teinture des cheveux humains vivants;
- des dérivés organiques des nitrites, tels que par exemple le nitrite d'amyle;
- des vecteurs de nitrites, c'est-à-dire des composés qui par transformation génèrent un nitrite du type défini ci-dessus.

Les nitrites particulièrement préférés sont les nitrites de sodium, de potassium ou d'ammonium.

Cette variante du procédé est mise en oeuvre an appliquant sur les matières kératiniques la composition (A) à base du colorant de formule (I) définie ci-dessus, puis une composition aqueuse acide (B), la composition (A) ou (B) contenant au moins un nitrite.

Les nitrites sont généralement utilisés dans des proportions comprises entre 0,02 et 1 mole/litre.

Selon une troisième variante de l'invention, les oxydants choisis parmi l'acide periodique et ses sels hydrosolubles, l'hypochlorite de sodium, le ferricyanure de potassium, l'oxyde d'argent, le réactif de Fenton, l'oxyde de plomb (IV), le sulfate de cesium, le persulfate d'ammonium, sont de préférence appliqués sur les fibres au moyen d'une composition (B) et après l'application de la composition (A).

Les oxydants particulièrement préférés de ce groupe sont l'acide periodique et ses sels hydrosolubles, tels que les sels de lithium, sodium, potassium, rhubidium, cesium, magnésium, calcium, strontium, manganèse, fer, cuivre, zinc, aluminium, les sels de sodium et de potassium étant particulièrement préférés.

Ces oxydants sont présents dans des proportions suffisantes pour développer une coloration, de préférence entre 0,004 mole et 0,07 mole et en particulier entre 0,01 mole et 0,04 mole pour 100 g de composition.

Selon une quatrième variante de l'invention, on applique dans un premier temps, sur les fibres kératiniques, une composition contenant, dans un milieu approprié pour la teinture, à un pH compris entre 2 et 10, un anion d'un métal ayant une bonne affinité pour la kératine, et ayant un potentiel d'oxydoréduction supérieur à celui des composés de formule (I). Cet anion est choisi de préférence parmi les permanganates ou les bichromates et plus particulièrement le permanganate de potassium et le bichromate de sodium.

Dans un second temps, on applique une composition contenant dans un milieu approprié pour la teinture, à un pH compris entre 4 et 10, un colorant répondant à la formule (I) définie ci-dessus.

Ces anions métalliques sont généralement utilisés à des molalités en anions supérieures à $10^{-3}$ moles/1000 g jusqu'à de préférence 1 mole/1000 g. Les compositions contenant les anions ne doivent pas contenir d'agents organiques ayant un effet réducteur sur ceux-ci.

Selon une cinquième variante de l'invention, on met en oeuvre des catalyseurs d'oxydation choisis parmi les sels métalliques, tels que des sels de manganèse, de cobalt, de fer, de cuivre et d'argent. A titre d'exemple, on peut citer le sulfate de manganèse, le lactate de manganèse, le chlorure de cobalt, le chlorure ferrique, le chlorure cuivrique, le nitrate d'argent ammoniacal. Les sels préférés sont les sels de cuivre. Ces sels sont utilisés dans des proportions de 0,01 à 2% exprimées en ion métallique.

Selon cette variante, on met les fibres kératiniques et en particulier les cheveux, en contact avec une composition (B) contenant dans un milieu approprié pour la teinture, le sel métallique avant ou après l'application de la composition (A) contenant le composé de formule (I) et on rince de préférence entre les deux étapes.

La forme de réalisation préférée consiste à appliquer un sel cuivrique dans un premier temps et la composition (A) dans un deuxième temps.

On peut faire suivre cette teinture, après rinçage, de l'application d'une solution de peroxyde d'hydrogène pour éventuellement éclaircir la couleur obtenue.

Selon une sixième variante, on utilise des sels de terres rares. Les sels de terres rares utilisables, conformément à l'invention, sont choisis parmi les sels de Lanthanides, et notamment les sels de Cérium $Ce^{3+}$, $Ce^{4+}$, de Lanthane $La^{3+}$, d'Europium $Eu^{2+}$, $Eu^{3+}$, de Gadolinium $Gd^{3+}$, d'Ytterbium $Yb^{2+}$, $Yb^{3+}$, de Dysprosium $Dy^{3+}$. Les sels préférés sont en particulier les sulfates, chlorures ou nitrates.

Ces sels de terres rares sont présents dans des proportions comprises entre 0,1 et 8% en poids par rapport au poids total de la composition.

On utilise de préférence les sels de Cérium $Ce^{3+}$ et $Ce^{4+}$ sous la forme de sulfates et de chlorures.

Lorsque des compositions à base de peroxyde d'hydrogène sont utilisées, la teneur en peroxyde d'hydrogène est généralement comprise entre 1 et 40 volumes et de préférence entre 2 et 10 volumes et plus particulièrement entre 3 et 10 volumes.

Selon une autre forme de réalisation, on applique, dans un premier temps, une composition tinctoriale renfermant dans un milieu cosmétiquement acceptable aqueux, ayant un pH inférieur ou égal à 7, au moins un dérivé indolique répondant à la formule :

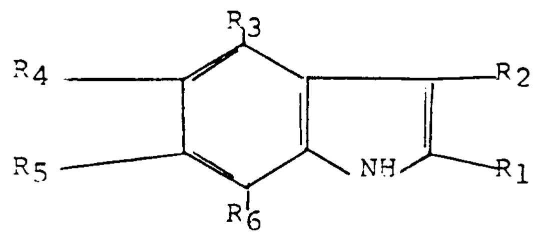

(II)

dans laquelle :

$R_1$, $R_2$, indentiques ou différents, désignent H ou $CH_3$;

$R_3$ désigne H, $NH_2$, OH, $-OCH_3$;

$R_4$ désigne H, $NH_2$, OH, $-OC_2H_5$;

$R_5$ désigne H, $NH_2$, OH, $-NHCH_2CH_2OH$;

$R_6$ désigne H, OH;

deux au moins des radicaux $R_3$, $R_4$, $R_5$ et $R_6$ désignant hydrogène, l'un au moins et un seul des groupements $R_3$, $R_4$ ou $R_5$ désignant $NH_2$ ou $-NHCH_2CH_2OH$ pour $R_5$; et lorsque $R_5$ désigne un groupement amino et $R_4$ un groupement OH, $R_1$ et $R_2$ désignent $CH_3$,

et les sels d'acides correspondants.

Après un temps de pose suivi d'un rinçage et d'un essorage, on applique, dans un second temps, une solution oxydante alcaline, cette application étant suivie d'un rinçage et d'un shampooing.

La demanderesse a constaté que ce procédé permettait d'obtenir des nuances variées à reflets ou des nuances naturelles de blonds clairs à foncés, de cuivre et d'acajou qui étaient uniformes même après plusieurs superpositions et couvraient bien les cheveux.

Les colorants plus particulièrement préférés dans cette forme de réalisation, sont choisis parmi le 4-aminoindole, le 5-aminoindole, le 6-aminoindole, le 6-amino 2,3-diméthyl 5-hydroxyindole, le 6-$\beta$-hydroxyéthylaminoindole.

La quantité d'amino indoles répondant à la formule (I) utilisée dans la composition (A), conforme à l'invention, est comprise généralement dans des proportions entre 0,01 et 5% en poids par rapport au poids total de la composition, et de préférence entre 0,03 et 2,5% en poids.

Les compositions utilisables, conformément à l'invention, peuvent se présenter sous des formes diverses, telles que sous forme de lotions plus ou moins épaissies, de crèmes, de mousses, de gels. Ces compositions peuvent également être présentées dans des dispositifs à plusieurs compartiments ou kits contenant les différents composants destinés à être mélangés ou moment de l'emploi ou encore sous la forme d'aérosols.

Les compositions utilisables dans le procédé conforme à l'invention et qui constituent également un objet de l'invention, sont caractérisées par le fait qu'elles contiennent dans un milieu approprié pour la teinture, au moins un dérivé d'amino indole de formule (I) et au moins des ions iodure ou des nitrites, tels que définis ci-dessus.

Les proportions des différents constituants sont telles que définies ci-dessus.

Le milieu approprié pour la teinture est de préférence un milieu aqueux qui doit être cosmétiquement acceptable lorsque les compositions sont destinées à la teinture des cheveux humains vivants. Ce milieu aqueux peut être constitué par de l'eau ou un mélange eau/solvant(s).

Les solvants sont choisis parmi les solvants organiques et préférentiellement parmi l'alcool éthylique, l'alcool propylique ou isopropylique, l'alcool tertiobutylique, l'éthylèneglycol, les éthers monométhylique, monoéthylique et monobutylique de l'éthylèneglycol, l'acétate du monoéthyléther de l'éthylèneglycol, le propylèneglycol, les monométhyl éthers du propylèneglycol et du dipropylèneglycol, le lactate de méthyle.

Les solvants particulièrement préférés sont l'alcool éthylique, le propylèneglycol et l'éther monobutylique de l'éthylèneglycol.

Selon une autre forme de réalisation, le milieu approprié pour la teinture peut être constitué par des solvants anhydres, tels que ceux définis préférentiellement ci-dessus, la composition étant dans ce cas, soit mélangée au moment de l'emploi avec un milieu aqueux, soit appliquée sur les fibres kératiniques mouillées au préalable par une composition aqueuse. On appelle, conformément à l'invention, un milieu solvant anhydre, un milieu contenant moins de 1% d'eau.

Le pH de la composition (A), lorsque le milieu approprié pour la teinture est aqueux, est compris de préférence entre 2 et 7 et en particulier entre 3,5 et 7.

Lorsque le milieu approprié pour la teinture est constitué par un mélange eau/solvant(s), les solvants sont utilisés dans des concentrations comprises entre 0,5 et 75% en poids, par rapport au poids total de la composition, de préférence entre 2 et 50% et plus particulièrement entre 2 et 20% en poids.

La composition colorante peut renfermer, outre le colorant indolique répondant à la formule (I) ou (II) ci-dessus, d'autres indoles parmi lesquels on peut citer le 2-méthyl 5,6-dihydroxyindole et son sel d'addition acide correspondant, le 5,6-dihydroxyindole et le 5,6-dihydroxy 2-carboxyindole.

Les compositions conformes à l'invention peuvent contenir tous autres adjuvants habituellement utilisés pour la teinture des fibres kératiniques et en particulier des adjuvants cosmétiquement acceptables, dans la mesure où ces compositions sont appliquées pour teindre les cheveux humains vivants.

Dans ce dernier cas, les compositions peuvent contenir notamment des amides gras dans des proportions préférentielles de 0,05 à 10% en poids, des agents tensio-actifs anioniques, cationiques, non ioniques, amphotères ou leurs mélanges présents de préférence dans des proportions comprises entre 0,1 et 50% en poids, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques.

Les épaississants sont choisis parmi l'alginate de sodium, la gomme arabique, la gomme de guar, les hétérobiopolysaccharides, tels que la gomme de xanthane, les scléroglucanes, les dérivés de cellulose, tels que la méthylcellulose, l'hydroxyéthylcellulose, l'hydroxypropylméthylcellulose, le sel de sodium de la carboxyméthylcellulose et les polymères d'acide acrylique préférentiellement réticulés.

On peut également utiliser des agents épaississants minéraux, tels que la bentonite. Ces épaississants sont utilisés seuls ou en mélange et sont présents de préférence dans des proportions comprises entre 0,1 et 5% en poids par rapport au poids total de la composition et avantageusement entre 0,5 et 3% en poids.

Les agents alcalinisants utilisables dans les compositions, peuvent être en particulier des amines, telles que les alcanolamines, des alkylamines, des hydroxydes ou carbonates alcalins ou d'ammonium.

Les agents d'acidification utilisables dans ces compositions peuvent être choisis parmi l'acide lactique, l'acide acétique, l'acide tartrique, l'acide phosphorique, l'acide chlorhydrique, l'acide citrique.

Il est bien entendu possible d'utiliser tout autre agent alcalinisant ou acidifiant acceptable, notamment dans le cas de la teinture des cheveux en cosmétique.

Lorsque les compositions sont utilisées sous forme de mousse, elles peuvent être conditionnées sous pression et dans un dispositif aérosol en présence d'un agent propulseur et d'au moins un générateur de mousse.

Les agents générateurs de mousse peuvent être des polymères moussants anioniques, cationiques, non ioniques, amphotères ou leurs mélanges ou des agents tensio-actifs du type de ceux définis ci-dessus.

Dans la forme de réalisation mettant en oeuvre les colorants de formule (II), la composition oxydante est constituée de préférence par une solution aqueuse d'agent oxydant que l'on mélange au moment de l'emploi avec une solution aqueuse alcaline pouvant renfermer des solvants et des agents tensio-actifs.

L'agent oxydant est choisi parmi le peroxyde d'hydrogène, le peroxyde d'urée, les persels tels que les percarbonates, les perborates alcalins ou d'ammonium.

Les agents alcalins sons choisis parmi l'ammoniaque, les alcanolamines telles que le 2-amino 2-méthyl 1-propanol, la monoéthanolamine, la monométhyléthanolamine ou la diméthyléthanolamine.

La solution aqueuse d'agent oxydant peut renfermer des solvants du type défini ci-dessus, des agents tensio-actifs ainsi que des cires auto-émulsionnables ou des alcools polyoxyéthylénés pour épaissir les solutions.

La proportion d'agent oxydant dans les compositions est comprise entre 1 et 15% en poids par rapport au poids total de la composition oxydante et de préférence entre 1 et 8%.

La proportion d'agent oxydant dans les compositions oxydantes alcalines que l'on applique sur les cheveux, est comprise entre 1 et 10% en poids par rapport au poids total de la composition et de préférence entre 1 et 5%.

Le pH de la composition oxydante est compris entre 8,5 et 12.

L'invention a également pour objet un agent de coloration des fibres kératiniques et en particulier des fibres kératiniques humaines, à plusieurs composants, dont l'un des composants est constitué par la

composition (A) définie ci-dessus et l'autre composant est constitué par l'une des compositions (B) également définie(s) ci-dessus, les composants respectifs étant choisis selon les différentes variantes exposées ci-dessus.

Un autre objet de l'invention est constitué par un dispositif à plusieurs compartiments encore appelé "kit de teinture" ou "nécessaire de teinture", comportant tous les composants destinés à être appliqués pour une même teinture sur les fibres kératiniques, en applications successives avec ou sans prémélange, comme mentionné ci-dessus.

De tels dispositifs sont connus en eux-mêmes et peuvent comporter un premier compartiment contenant la composition (A) qui comprend le dérivé d'amino indole de formule (I) dans un milieu approprié pour la teinture et un second compartiment qui contient une composition (B) définie ci-dessus.

Lorsque le milieu contenant le dérivé d'amino indole de formule (I) est anhydre, on peut prévoir un troisième compartiment contenant un milieu aqueux approprié pour la teniture destiné à être mélangé avant l'emploi avec la composition de ce premier compartiment.

Les dispositifs à plusieurs compartiments utilisables conformément à l'invention, peuvent être équipés de moyens de mélange au moment de l'emploi et leur contenu peut être conditionné sous atmosphère inerte.

Le procédé et les compositions utilisés, conformément à l'invention, peuvent être mis en oeuvre pour teindre les cheveux naturels ou déjà teints, permanentés ou non, défrisés ou non, ou les cheveux fortement ou légèrement décolorés, éventuellement permanentés. Il est également possible de les utiliser pour le teinture de la fourrure ou de la laine.

Les exemples qui suivent sont destinés à illustrer l'invention.

EXEMPLE DE PREPARATION 1

Préparation du 6-N-β-hydroxyéthylamino indole.

Etape 1

Préparation du 6-N-(β-chloroéthoxycarbonyl)amino indole.

On chauffe au reflux 0,05 mole (6,6 g) de 6-amino indole, 5,5 g de carbonate de calcium dans 30 ml de dioxane. On ajoute peu à peu 0,055 mole (7,9 g) de chloroformiate de β-chloréthyle. Le mélange réactionnel est dilué à la glace. Le produit attendu précipite. Il fond à 134°C.

L'analyse du produit recristallisé de l'éthanol donne les résultats suivants :

| Analyse pour $C_{11}H_{11}N_2O_2Cl$ | | | | | |
|---|---|---|---|---|---|
| | C | H | Cl | O | N |
| Calculé<br>Trouvé | 55,36<br>55,40 | 4,65<br>4,68 | 14,85<br>14,72 | 13,41<br>13,27 | 11,74<br>11,67 |

Etape 2

Préparation du 6-N-β-hydroxyéthylamino indole.

On ajoute 0,28 mole (66,5 g) de 6-N-(β-chloroéthoxycarbonyl)amino indole à 200 ml de soude 4N et 66,5 ml d'éthanol. Le mélange réactionnel est chauffé 1 heure au reflux. On précipite le produit attendu par ajout de glace. Il fond à 99°C.

L'analyse élémentaire du produit obtenu donne les résultats suivants :

| Analyse pour $C_{10}H_{12}N_2O$ | | | | |
|---|---|---|---|---|
| | C | H | N | O |
| Calculé | 68,16 | 6,86 | 15,90 | 9,08 |
| Trouvé | 67,88 | 6,91 | 15,91 | 9,15 |

EXEMPLE DE PREPARATION 2

Préparation du chlorhydrate de 6-N-(β-hydroxyéthyl)amino 1-méthyl indole.

Etape 1

Préparation de la N-(6-indolyl)oxazolidine-1,3 one-2

A 120 ml d'une solution de méthylate de sodium à 30% dans le méthanol, on ajoute 60 ml de méthanol puis, sous agitation, 0,25 mole (60 g) de 6-(β-chloréthoxycarbonyl)amino indole (préparé selon la première étape de l'exemple 1). La température atteint 50 °C. L'agitation est maintenue 15 minutes après la fin de l'addition. Le précipité formé est essoré, lavé à l'alcool puis séché. Il fond à 199 °C.

L'analyse du produit recristallisé de l'acide acétique donne les résultats suivants :

| Analyse pour $C_{11}H_{10}N_2O_2$ | | | | |
|---|---|---|---|---|
| | C | H | N | O |
| Calculé | 65,34 | 4,98 | 13,85 | 15,82 |
| Trouvé | 65,42 | 5,02 | 13,75 | 15,86 |

Etape 2

Préparation de la N-[-6(1-méthyl)indolyl]oxazolidine-1,3 one-2

A une solution de 0,15 mole (30,5 g) de N-(6-indolyl)oxazolidine-1,3 one-2 dans 300 ml de diméthylformamide, on ajoute 100 ml d'une solution à 30% de méthylate de sodium dans le méthanol. Le mélange réactionnel est chauffé à 40 °C. On ajoute goutte à goutte 28 ml d'iodure de méthyle. Le chauffage est maintenu 1 heure après la fin de l'addition. Après dilution du milieu réactionnel par de l'eau glacée, le produit attendu précipite. Après essorage, lavage à l'eau puis à l'éthanol, il fond à 160 °C.

L'analyse du produit recristallisé de l'acide acétique donne les résultats suivants :

| Analyse pour $C_{12}H_{12}N_2O_2$ | | | | |
|---|---|---|---|---|
| | C | H | N | O |
| Calculé | 66,65 | 5,59 | 12,95 | 14,80 |
| Trouvé | 66,55 | 5,62 | 12,76 | 15,08 |

Etape 3

Préparation du chlorhydrate de 6-(β-hydroxyéthyl)amino 1-méthyl indole.

On chauffe 1 heure au reflux 0,02 mole (4,32 g) de N-[6-(1-méthyl)indolyl]oxazolidine-1,3 one-2 dans 17 ml de soude 4N additionnée de 2 ml d'éthanol. Le milieu réactionnel est dilué par de l'eau glacée, puis le produit obtenu est extrait par l'acétate d'éthyle.

L'huile obtenue après évaporation de l'acétate d'éthyle est ajoutée à 7 ml d'une solution d'acide chlorhydrique 7M dans l'éthanol. Le produit attendu précipite.

L'analyse du produit obtenu après lavage et séchage, donne les résultats suivants :

| Analyse pour $C_{11}H_{15}ClN_2O$ | | | | | |
|---|---|---|---|---|---|
| | C | H | Cl | N | O |
| Calculé | 58,28 | 6,67 | 15,64 | 12,36 | 7,06 |
| Trouvé | 58,16 | 6,70 | 15,52 | 12,45 | 7,13 |

EXEMPLE DE PREPARATION 3

Préparation du chlorhydrate de 6-amino 2,3,4-triméthyl indole.

Etape 1

Préparation du 3-méthyl 5-nitro-acétanilide

A une solution de 12,2 g de 3-méthyl 5-nitro-aniline dans 36 ml d'acétate d'éthyle au reflux, on ajoute goutte à goutte 9 g d'anhydride acétique. Après 30 minutes au reflux, on refroidit, on essore le précipité, on lave avec de l'acétate d'éthyle (10 ml) et on sèche le produit. On obtient 15,2 g de produit attendu.

L'analyse du produit recristallisé de l'éthanol donne les résultats suivants :

PF = 185 °C.

| Analyse pour $C_9H_{10}N_2O_3$ | | | | |
|---|---|---|---|---|
| | C | H | N | O |
| Calculé | 55,67 | 5,19 | 14,43 | 24,72 |
| Trouvé | 55,66 | 5,20 | 14,40 | 24,83 |

Etape 2

Préparation du 3-acétamido 5-méthylaniline.

A 2,6 g de Pd/C à 10% mouillé avec 2,6 g d'eau sont ajoutés 40 ml d'éthanol, 25 ml de cyclohexène, puis 12,6 g de 3-méthyl 5-nitro-acétanilide. Après 2 heures de reflux, la suspension est filtrée à chaud, le solide est lavé à l'éthanol (50 ml). Le filtrat est évaporé à sec sous vide. On obtient 10 g de précipité blanc.

L'analyse du produit recristallisé de l'acétate d'éthyle donne les résultats suivants :

PF = 123 °C.

| Analyse pour $C_9H_{12}N_2O$ | | | | |
|---|---|---|---|---|
| | C | H | N | O |
| Calculé | 65,83 | 7,37 | 17,06 | 9,74 |
| Trouvé | 65,40 | 7,35 | 16,94 | 10,69 |

Etape 3

Préparation du 6-acétamido 2,3,4-triméthyl indole.

On dissout 9 g de 3-acétamido 5-méthyl-aniline dans 20 ml de diméthylformamide, puis on ajoute 3,2 ml de 3-bromo butanone-2. On laisse 1 heure à la température ambiante, puis on porte la température à 100-110 °C pendant une heure. On refroidit, on jette dans 100 ml d'eau glacée. On essore le précipité, lave successivement avec 50 ml d'eau distillée, 20 ml d'éthanol, 20 ml d'éther isopropylique. On obtient 4,2 g

de précipité blanc.

L'analyse du produit recristallisé de l'acide acétique donne les résultats suivants :

PF = 255 ° C

| Analyse pour $C_{13}H_{16}N_2O$ | | | | |
|---|---|---|---|---|
| | C | H | N | O |
| Calculé<br>Trouvé | 72,19<br>71,04 | 7,46<br>7,52 | 12,95<br>12,73 | 7,40<br>8,42 |

Etape 4

Préparation du 6-amino 2,3,4-triméthyl indole.

Une suspension de 3,67 g de 6-acétamido 2,3,4-triméthyl indole dans 15 ml d'acide chlorhydrique (12N) est portée à 100 ° C pendant 3 heures. On refroidit, essore le précipité, lave successivement par 3 ml d'acide chlorhydrique (12N), 20 ml d'éthanol absolu. On obtient 3,5 g d'un précipité blanc.

L'analyse du produit donne les résultats suivants :

| Analyse pour $C_{11}H_{15}$ Cl $N_2$ | | | | |
|---|---|---|---|---|
| | C | H | Cl | N |
| Calculé<br>Trouvé | 62,70<br>62,72 | 7,18<br>7,24 | 16,83<br>17,09 | 13,29<br>13,22 |

EXEMPLE DE PREPARATION 4

Préparation du chlorhydrate de 6-amino 2,3,7-triméthyl indole.

Etape 1

Préparation du 2-méthyl 3-nitro-acétanilide

On opère d'une manière identique à celle de l'étape 1 de l'exemple 3 à partir du 2-méthyl 3-nitro-aniline.

L'analyse du produit recristallisé de l'éthanol donne les résultats suivants :

PF = 146 ° C reprise puis 162 ° C

| Analyse pour $C_9H_{10}N_2O_3$ | | | | |
|---|---|---|---|---|
| | C | H | N | O |
| Calculé<br>Trouvé | 55,67<br>55,72 | 5,19<br>5,27 | 14,43<br>14,42 | 24,72<br>24,97 |

Etape 2

Préparation du 2-méthyl 3-acétamido-aniline

On opère d'une manière identique à celle de l'etape 2 de l'exemple 3.

L'analyse du produit recristallisé d'un mélange d'acétate d'éthyle/éthanol (5/2) donne les résultats suivants :

PF = 145 ° C

| Analyse pour $C_9H_{12}N_2O$ | | | | |
|---|---|---|---|---|
| | C | H | N | O |
| Calculé | 65,83 | 7,37 | 17,06 | 9,74 |
| Trouvé | 65,76 | 7,44 | 17,18 | 9,71 |

Etape 3

Préparation du 6-acétamido 2,3,7-triméthyl indole.

On opère d'une manière identique à celle de l'étape 3 de l'exemple 3.
L'analyse du produit recristallisé de l'acide acétique donne les résultats suivants :
PF = 242 °C

| Analyse pour $C_{13}H_{16}N_2O$ | | | | |
|---|---|---|---|---|
| | C | H | N | O |
| Calculé | 72,19 | 7,46 | 12,95 | 7,40 |
| Trouvé | 72,16 | 7,46 | 12,88 | 7,25 |

Etape 4

Préparation du chlorhydrate de 6-amino 2,3,7-triméthyl indole.

On opère d'une manière identique à celle de l'étape 4 de l'exemple 3.
L'analyse du produit donne les résultats suivants :

| Analyse pour $C_{11}H_{15}ClN_2$ | | | | |
|---|---|---|---|---|
| | C | H | Cl | N |
| Calculé | 62,70 | 7,18 | 16,83 | 13,29 |
| Trouvé | 62,72 | 7,18 | 17,02 | 13,31 |

EXEMPLE DE PREPARATION 5

Préparation du chlorhydrate de 6-amino 2,3,4,5-tétraméthyl indole.

Etape 1

Préparation du 2,3-diméthyl 5-nitro-acétanilide

On opère d'une manière identique à celle de l'étape 1 de l'exemple 3, à partir du 2,3-diméthyl 5-nitro-aniline.
L'analyse du produit recristallisé de l'éthanol donne les résultats suivants :
PF = 230 °C

| Analyse pour $C_{10}H_{12}N_2O_3$ | | | | |
|---|---|---|---|---|
| | C | H | N | O |
| Calculé | 57,69 | 5,81 | 13,45 | 23,05 |
| Trouvé | 57,77 | 5,90 | 13,40 | 23,12 |

Etape 2

Préparation du 3-acétamido 4,5-diméthyl aniline

On opère d'une manière identique à celle de l'étape 2 de l'exemple 3.
L'analyse du produit recristallisé de l'éthanol donne les résultats suivants :
PF = 162°C

| Analyse pour $C_{10}H_{14}N_2O$ | | | | |
|---|---|---|---|---|
| | C | H | N | O |
| Calculé | 67,39 | 7,92 | 15,72 | 8,98 |
| Trouvé | 67,38 | 7,98 | 15,63 | 9,08 |

Etape 3

Préparation du 6-acétamido 2,3,4,5-tétraméthyl indole

On opère d'une manière identique à celle de l'étape 3 de l'exemple 3.
L'analyse du produit recristallisé de l'acide acétique donne les résultats suivants :
PF = 265°C

| Analyse pour $C_{14}H_{18}N_2O$ | | | | |
|---|---|---|---|---|
| | C | H | N | O |
| Calculé | 73,01 | 7,88 | 12,16 | 6,95 |
| Trouvé | 72,93 | 7,87 | 12,19 | 7,07 |

Etape 4

Préparation du chlorhydrate de 6-amino 2,3,4,5-tétraméthyl indole

On opère d'une manière identique à celle de l'étape 4 de l'exemple 3.
L'analyse du produit donne les résultats suivants :

| Analyse pour $C_{12}H_{17}Cl\ N_2$ | | | | |
|---|---|---|---|---|
| | C | H | Cl | N |
| Calculé | 64,13 | 7,62 | 15,78 | 12,47 |
| Trouvé | 64,03 | 7,71 | 15,83 | 12,50 |

EXEMPLE DE PREPARATION 6

Préparation du chlorhydrate de 6-amino 2,3-diméthyl 5-éthyl indole

Etape 1

Préparation du 2-éthyl 5-nitro-acétanilide

On opère d'une manière identique à celle de l'étape 1 de l'exemple 3, à partir du 2-éthyl 5-nitro-aniline. L'analyse du produit recristallisé de l'éthanol donne les résultats suivants :
PF = 158°C

| Analyse pour $C_{10}H_{12}N_2O_3$ | | | | |
|---|---|---|---|---|
| | C | H | N | O |
| Calculé | 57,69 | 5,81 | 13,45 | 23,05 |
| Trouvé | 57,63 | 5,82 | 13,43 | 22,98 |

Etape 2

Préparation du 3-acétamido 4-éthyl-aniline

On opère d'une manière identique à celle de l'etape 2 de l'exemple 3. L'analyse du produit recristallisé de l'éthanol donne les résultats suivants :
PF = 150°C

| Analyse pour $C_{10}H_{14}N_2O$ | | | | |
|---|---|---|---|---|
| | C | H | N | O |
| Calculé | 67,39 | 7,92 | 15,72 | 8,98 |
| Trouvé | 67,44 | 7,93 | 15,89 | 9,06 |

Etape 3

Préparation du 6-acétamido 2,3-diméthyl 5-éthyl indole

On opère d'une manière identique à celle de l'étape 3 de l'exemple 3. L'analyse du produit recristallisé de l'acide acétique donne les résultats suivants :
PF = 251°C

| Analyse pour $C_{14}H_{18}N_2O$ | | | | |
|---|---|---|---|---|
| | C | H | N | O |
| Calculé | 73,01 | 7,88 | 12,16 | 6,95 |
| Trouvé | 73,02 | 7,88 | 12,07 | 6,97 |

Etape 4

Préparation du chlorhydrate de 6-amino 2,3-diméthyl 5-éthyl indole

On opère d'une manière identique à celle de l'étape 4 de l'exemple 3. L'analyse du produit donne les résultats suivants :

| Analyse pour $C_{12}H_{17}Cl\ N_2$ | | | | |
|---|---|---|---|---|
| | C | H | Cl | N |
| Calculé<br>Trouvé | 64,13<br>73,97 | 7,62<br>7,70 | 15,78<br>15,58 | 12,47<br>12,55 |

EXEMPLE DE PREPARATION 7

Préparation du chlorhydrate de 6-amino 5-chloro 2,3-diméthyl indole.

Etape 1

Préparation du 2-chloro 5-nitro-acétanilide

On opère d'une manière identique à celle de l'étape 1 de l'exemple 3 à partir du 2-chloro 5-nitro aniline.

L'analyse du produit recristallisé de l'éthanol donne les résultats suivants :

PF = 157°C

| Analyse pour $C_8H_7Cl\ N_2O_3$ | | | | | |
|---|---|---|---|---|---|
| | C | H | Cl | N | O |
| Calculé<br>Trouvé | 44,77<br>44,87 | 3,29<br>3,30 | 16,52<br>16,44 | 13,05<br>13,10 | 22,37<br>22,24 |

Etape 2

Préparation du 3-acétamido 4-chloro-aniline

On porte une suspension de 345 g de fer dans un mélange de 850 ml d'eau et 20 ml d'acide acétique à 90°C et on ajoute en 45 minutes 172,5 g de 2-chloro 5-nitro-acétanilide. Après 10 minutes à 95°C, on refroidit, filtre les boues ferriques, on lave trois fois celles-ci avec 1 litre d'acétone. Le filtrat est évaporé à sec pour conduire à 112 g de précipité blanc.

L'analyse du produit recristallisé de l'acétone donne les résultats suivants :

PF = 198°C

| Analyse pour $C_8H_9Cl\ N_2O$ | | | | | |
|---|---|---|---|---|---|
| | C | H | Cl | N | O |
| Calculé<br>Trouvé | 52,05<br>52,10 | 4,91<br>4,94 | 19,2<br>19,45 | 15,17<br>15,24 | 8,67<br>8,78 |

Etape 3

Préparation du 6-acétamido 5-chloro 2,3-diméthyl indole

On opère d'une manière identique à celle de l'étape 3 de l'exemple 3.

L'analyse du produit recristallisé de l'acide acétique donne les résultats suivants :

PF = 264°C

| Analyse pour $C_{12}H_{13}Cl\ N_2O$ | | | | | |
|---|---|---|---|---|---|
| | C | H | Cl | N | O |
| Calculé<br>Trouvé | 60,89<br>60,78 | 5,54<br>5,53 | 14,98<br>15,15 | 11,83<br>11,86 | 6,76<br>6,77 |

Etape 4

Préparation du chlorhydrate de 6-amino 5-chloro 2,3-diméthyl indole

On opère d'une manière identique à celle de l'étape 4 de l'exemple 3.

L'analyse du produit donne les résultats suivants :

| Analyse pour $C_{10}H_{12}Cl\ N_2$ | | | | |
|---|---|---|---|---|
| | C | H | Cl | N |
| Calculé<br>Trouvé | 51,97<br>52,00 | 5,23<br>5,29 | 30,68<br>30,63 | 12,12<br>11,88 |

EXEMPLE DE PREPARATION 8

Préparation du 6-N-($\beta$,$\gamma$-dihydroxypropyl)amino indole.

On dissout 26,4 g de 6-amino indole dans 70 ml d'alcool absolu. On ajoute 29,6 g de glycidol et on agite pendant 4 heures à 30-40 ° C.

On verse sur 200 g d'eau glacée et on extrait par trois fois 100 ml d'acétate d'éthyle. On lave le solvant à l'eau. On le sèche sur $Na_2SO_4$ et chasse à sec sous vide.

Le résidu huileux est repris trois fois dans 0,6 litre d'éther isopropylique au reflux. On filtre l'éther et chasse à sec sous vide. On reprend l'huile résiduelle dans 100 $cm^3$ d'acétate d'éthyle et on purifie sur colonne de silice (éluant acétate d'éthyle 9/Heptane 1).

La fraction contenant le produit attendu est évaporée à sec sous vide.

On obtient une huile incolore qui donne les résultats suivants :

| Analyse pour $C_{11}H_{14}N_2O_2$ | | | | |
|---|---|---|---|---|
| | C | H | N | O |
| Calculé<br>Trouvé | 64,06<br>63,95 | 6,84<br>6,98 | 13,58<br>13,48 | 15,51<br>15,59 |

EXEMPLES 1 à 4

On prépare les compositions suivantes :

| | |
|---|---|
| - Colorant indolique | xg |
| - Iodure de potassium | yg |
| - Alcool éthylique | 10,0 g |
| - Gomme de guar vendue sous la dénomination de JAGUAR HP60 par la Société MEYHALL | 1,0 g |
| - Alkyléther de glycoside vendu à la concentration de 60% MA sous la dénomination de TRITON CG110 par la Société ROHM & HAAS | 5,0 g MA |
| - Conservateur | 0,6 g |
| - Eau déminéralisée | qsp 100,0 g |

Le pH est ajusté à la valeur indiquée dans le tableau par ajout d'agent alcalinisant ou acidifiant dénommé "agent de pH".

| EXEMPLE | COMPOSE | X | Y | AGENT de pH | pH |
|---|---|---|---|---|---|
| 1 | 6-amino indole | 1,0 | 0,9 | Acide citrique | 6,5 |
| 2 | 7-amino indole | 1,0 | 0,9 | Triéthanolamine | 6,5 |
| 3 | 4-amino indole | 0,6 | 0,4 | Acide citrique | 6,5 |
| 4 | 5-amino indole | 0,5 | 0,45 | Acide citrique | 6,5 |

Les compositions 1 à 4 sont appliquées 15 minutes sur des cheveux gris à 90% de blancs. Après rinçage intermédiaire, on applique pendant 5 minutes un lait oxydant à pH 3 titrant 12,5 volumes en eau oxygénée. On rince à nouveau et on effectue un shampooing final. On sèche.

Les nuances obtenues sont les suivantes :

| COMPOSITION | COULEURS |
|---|---|
| 1 | châtain |
| 2 | blond très clair légèrement cendré |
| 3 | blond très clair légèrement cendré |
| 4 | blond clair nacré |

EXEMPLES 5 et 6

On prépare les compositions suivantes :

| | |
|---|---|
| - Colorant indolique | 1,0 g |
| - Iodure de potassium | 0,5 g |
| - Monobutyléther d'éthylèneglycol | z1 g |
| - Nonylphénol à 9 moles d'oxyde d'éthylène vendu sous la dénomination de SINNOPAL NP9 par la Société HENKEL | z2 g |
| - Eau déminéralisée | qsp 100,0 g |

| EXEMPLE | COMPOSE | Z1 | Z2 | AGENT de pH | pH |
|---|---|---|---|---|---|
| 5 | Chlorhydrate de 2,3-diméthyl 5-amino 6-méthoxy indole | 10,0 | 2,5 | Triéthanolamine | 5,5 |
| 6 | 2,3-diméthyl 5-méthoxy 6-amino indole | 17,5 | 5,0 | Acide lactique | 7,0 |

Les compositions 5 et 6 sont appliquées dans les mêmes conditions que les compositions 1 à 4.

Les nuances obtenues sont les suivantes :

| COMPOSITION | COULEURS |
|---|---|
| 5 | Blond naturel doré à tendance nacré |
| 6 | Blond clair nacré |

EXEMPLE 7

| | |
|---|---|
| - 6-amino indole | 1,0 g |
| - Alcool éthylique | 10,0 g |
| - Iodure de potassium | 1,0 g |
| - Dérivé de gomme de guar vendu sous la dénomination de JAGUAR HP 60 par la Société MEYHALL | 1,0 g |
| - Alkyléther de glycoside vendu sous la dénomination de TRITON CG 110 par la Société ROHM & HAAS | 5,0 g MA |
| - Conservateurs qs | |
| - pH spontané = 7,1 | |
| - Eau déminéralisée | qsp 100,0 g |

Cette composition est appliquée 15 minutes sur des cheveux gris à 90% de blancs. Après rinçage, on applique pendant 5 minutes un lait oxydant à pH 3 titrant 12,5 volumes en eau oxygénée. On rince, on effectue un shampooing et on sèche. On obtient en final une coloration châtain foncé.

EXEMPLE 8

COMPOSITION (A)

| | |
|---|---|
| - 6-amino indole | 0,5 g |
| - Monobutyléther de l'éthylèneglycol | 12,0 g |
| - Nonylphénol à 9 moles d'oxyde d'éthylène vendu sous la dénomination de SINNOPAL NP 9 par la Société HENKEL | 20,0 g |
| - Acide citrique qs pH = 5,2 | |
| - Eau déminéralisée | qsp 100,0 g |

COMPOSITION (B)

| | |
|---|---|
| - Métaperiodate de sodium | 3,5 g |
| - Alcool éthylique | 5,0 g |
| - Acide citrique qs pH = 4,0 | |
| - Eau déminéralisée | qsp 100,0 g |

On applique 20 minutes la composition (A) sur des cheveux gris à 90% de blancs. On rince, puis on applique 15 minutes la composition (B). On rince à nouveau. On obtient en final, après séchage, une coloration blond foncé cendré.

EXEMPLE 9

COMPOSITION (A)

| | |
|---|---|
| - 6-amino indole | 1,5 g |
| - Alcool éthylique | 6,5 g |
| - Propylèneglycol | 2,0 g |
| - Nonylphénol à 9 moles d'oxyde d'éthylène vendu sous la dénomination de SINNOPAL NP 9 par la Société HENKEL | 14,0 g |
| - Acide citrique qs pH = 4,5 | |
| - Eau déminéralisée | qsp 100,0 g |

COMPOSITION (B)

| | |
|---|---|
| - Nitrite de sodium | 2,0 g |
| - Acide chlorhydrique qs pH = 3,8 | |
| - Eau déminéralisée | qsp 100,0 g |

La composition (A) est appliquée 20 minutes sur des cheveux gris à 90% de blancs. On rince, puis on applique pendant 5 minutes la composition (B). On rince et on sèche. Les cheveux sont alors teints dans une nuance blond doré cuivré intense.

EXEMPLE 10

COMPOSITION (A)

| | |
|---|---|
| - Permanganate de potassium | 0,40 g |
| - Acide chlorhydrique qs pH = 3,0 | |
| - Eau déminéralisée | qsp 100,0 g |

COMPOSITION (B)

| | |
|---|---|
| - 6-amino indole | 2,0 g |
| - Monoéthyléther de l'éthylèneglycol | 12,0 g |
| - Polyéthylèneglycol de PM = 280 | 15,0 g |
| - Acide citrique qs pH = 5,0 | |
| - Eau déminéralisée | qsp 100,0 g |

Sur des cheveux gris à 90% de blancs, on applique pendant 15 minutes la composition (A). On rince, puis on applique la composition (B) pendant 20 minutes. On rince à nouveau et on sèche. La coloration finale obtenue est un châtain clair naturel.

EXEMPLE 11

COMPOSITION (A)

| | |
|---|---|
| - Sulfate de cuivre à 5 moles d'eau | 1,0 g |
| - Lauryléther sulfate de sodium à 2 moles d'oxyde d'éthylène vendu sous la dénomination de SACTIPON 8533 par la Société LEVER | 5,0 g MA |
| - Hydroxyéthyl cellulose vendue sous la dénomination de CELLOSIZE WP 3H par la Société UNION CARBIDE | 2,4 g MA |
| - Conservateurs qs | |
| - Monoéthanolamine qs pH = 9,5 | |
| - Eau déminéralisée | qsp 100,0 g |

COMPOSITION (B)

| | |
|---|---|
| - 6-amino indole | 2,2 g |
| - Alcool éthylique | 10,0 g |
| - Lauryléther sulfate de sodium à 2 moles d'oxyde d'éthylène vendu sous la dénomination de SACTIPON 8533 par la Société LEVER | 5,0 g MA |
| - Soude qs pH = 8,5 | |
| - Eau déminéralisée | qsp 100,0 g |

La composition (A) est appliquée 5 minutes sur des cheveux gris à 90% de blancs. On rince et on applique pendant 10 minutes la composition (B). On rince à nouveau et on sèche. Les cheveux sont alors teints dans une nuance blond foncé doré mat.

EXEMPLE 12

COMPOSITION (A)

| | |
|---|---|
| - Chlorure cerreux à 7 moles d'eau | 1,0 g |
| - Dérivé de gomme de guar vendu sous la dénomination de JAGUAR HP 60 par la Société MEYHALL | 0,42 g |
| - Conservateurs qs | |
| - Acide citrique qs pH = 4,5 | |
| - Eau déminéralisée | qsp 100,0 g |

COMPOSITION (B)

| | |
|---|---|
| - 6-amino indole | 1,5 g |
| - Monobutyléther de l'éthylèneglycol | 10,0 g |
| - Nonylphénol à 9 moles d'oxyde d'éthylène vendu sous la dénomination de SINNOPAL NP9 par la Société HENKEL | 15,0 g |
| - Acide citrique qs pH = 6,0 | |
| - Eau déminéralisée | qsp 100,0 g |

Sur des cheveux gris à 90% de blancs, on applique pendant 10 minutes la composition (A). On rince, puis on applique pendant 10 minutes la composition (B). Après un nouveau rinçage et un séchage, les

cheveux sont colorés dans une nuance blond très clair doré mat.

EXEMPLE 13

COMPOSITION (A)

| | |
|---|---|
| - Sulfate de cuivre à 5 moles d'eau | 1,0 g |
| - Lauryléther sulfate de sodium à 2 moles d'oxyde d'éthylène vendu sous la dénomination de SACTIPON 8533 par la Société LEVER | 5,0 g MA |
| - Hydroxyéthylcellulose vendue sous la dénomination de CELLOSIZE WP 3H par la Société UNION CARBIDE | 2,4 g MA |
| - Conservateurs qs | |
| - Monoéthanolamine qs pH = 9,5 | |
| - Eau déminéralisée | qsp 100,0 g |

COMPOSITION (B)

| | |
|---|---|
| - 5-amino indole | 0,5 g |
| - Alcool éthylique | 10,0 g |
| - Lauryléther sulfate de sodium à 2 moles d'oxyde d'éthylène vendu sous la dénomination de SACTIPON 8533 par la Société LEVER | 5,0 g MA |
| - Soude qs pH = 8,5 | |
| - Eau déminéralisée | qsp 100,0 g |

Des cheveux gris à 90% de blancs sont traités 5 minutes avec la composition (A). On rince, puis on applique 10 minutes la composition (B). On rince à nouveau et on sèche. Les cheveux sont alors teints dans une nuance blond doré mat.

EXEMPLE 14

COMPOSITION (A)

| | |
|---|---|
| - Permanganate de potassium | 0,4 g |
| - Acide chlorhydrique qs pH = 3 | |
| - Eau déminéralisée | qsp 100,0 g |

COMPOSITION (B)

| | |
|---|---|
| - 4-amino indole | 0,5 g |
| - Monobutyléther de l'éthylèneglycol | 12,0 g |
| - Nonylphénol à 9 moles d'oxyde d'éthylène vendu sous la dénomination de SINNOPAL NP9 par la Société HENKEL | 20,0 g |
| - Acide citrique qs pH = 5 | |
| - Eau déminéralisée | qsp 100,0 g |

On applique sur des cheveux gris à 90% de blancs, pendant 15 minutes, la composition (A). On rince, puis on applique la composition (B) pendant 20 minutes. On rince et on sèche. Les cheveux sont alors colorés dans une nuance blond foncé cendré.

EXEMPLE 15

COMPOSITION (A)

| | |
|---|---|
| - Dibromhydrate de 2,3-diméthyl 5-amino 6-hydroxyindole<br>- Monobutyléther de l'éthylèneglycol<br>- Nonylphénol à 9 moles d'oxyde d'éthylène vendu sous la dénomination de SINNOPAL NP9 par la Société HENKEL<br>- Triéthanolamine qs pH = 3,7<br>- Eau déminéralisée | 1,0 g<br>12,0 g<br>20,0 g<br><br><br>qsp 100,0 g |

COMPOSITION (B)

| | |
|---|---|
| - Métaperiodate de sodium<br>- Alcool éthylique<br>- Acide citrique qs pH = 4<br>- Eau déminéralisée | 3,5 g<br>5,0 g<br><br>qsp 100,0 g |

La composition (A) est appliquée 20 minutes sur des cheveux gris à 90% de blancs. Après rinçage, on applique 15 minutes la composition (B). On rince à nouveau et on sèche. On obtient en final un blond doré.

EXEMPLE 16

| | |
|---|---|
| - 6-N-$\beta$-hydroxyéthylamino indole<br>- Iodure de potassium<br>- Alcool éthylique<br>- Dérivé de gomme de guar vendu sous la dénomination de JAGUAR HP 60 par la Société MEYHALL<br>- Alkyléther de glycoside vendu sous la dénomination de TRITON CG 110 par la Société ROHM & HAAS<br>- Conservateurs qs<br>- pH sponané = 7,2<br>- Eau déminéralisée | 1,0 g<br>1,0 g<br>10,0 g<br>1,0 g<br><br>5,0 g MA<br><br><br><br>qsp 100,0 g |

Cette composition est appliquée 15 minutes sur des cheveux gris à 90% de blancs. On rince, puis on applique 5 minutes un lait oxydant à pH 3 titrant 12,5 volumes en eau oxygénée. Les cheveux en final sont teints dans une nuance blond foncé avec un reflet cendré très mat.

EXEMPLE 17

COMPOSITION (A)

| | |
|---|---|
| - 4-amino indole | 0,6 g |
| - Alcool éthylique | 10,0 g |
| - Dérivé de gomme de guar vendu sous la dénomination de JAGUAR HP 60 par la Société MEYHALL | 1,0 g |
| - Alkyléther de glycoside vendu sous la dénomination de TRITON CG 110 par la Société ROHM & HAAS | 5,0 g MA |
| - Conservateurs qs | |
| - pH spontané = 6,9 | |
| - Eau déminéralisée | qsp 100,0 g |

COMPOSITION (B)

| | |
|---|---|
| - Métaperiodate de sodium | 5,0 g |
| - Acide chlorhydrique qs pH = 3 | |
| - Eau déminéralisée | qsp 100,0 g |

On applique la composition (A) 15 minutes sur des cheveux gris à 90% de blancs. On rince, puis on applique la composition (B) pendant 15 minutes. On rince à nouveau puis on sèche. On obtient des cheveux colorés dans une nuance blond foncé cendré mat.

EXEMPLE 18

COMPOSITION (A)

| | |
|---|---|
| - 5-amino indole | 0,5 g |
| - Alcool éthylique | 10,0 g |
| - Dérivé de gomme de guar vendu sous la dénomination de JAGUAR HP 60 par la Société MEYHALL | 1,0 g |
| - Alkyléther de glycoside vendu sous la dénomination de TRITON CG 110 par la Société ROHM & HAAS | 5,0 g MA |
| - Conservateurs qs | |
| - pH spontané = 7,5 | |
| - Eau déminéralisée | qsp 100,0 g |

COMPOSITION (B)

| | |
|---|---|
| - Métaperiodate de sodium | 5,0 g |
| - Acide chlorhydrique qs pH = 3 | |
| - Eau déminéralisée | qsp 100,0 g |

La composition (A) est appliquée 15 minutes sur des cheveux gris à 90% de blancs. Les cheveux sont alors rincés, puis on applique 15 minutes la composition (B). On rince à nouveau puis on sèche. En final, les cheveux sont teints dans une nuance blond doré beige.

EXEMPLE 19

COMPOSITION (A)

| | |
|---|---|
| - 6-N-β-hydroxyéthylamino indole | 1,0 g |
| - Monoéthyléther de l'éthylèneglycol | 10,0 g |
| - Lauryléther sulfate de sodium à 2 moles d'oxyde d'éthylène vendu sous la dénomination de SACTIPON 8533 par la Société LEVER | 5,0 g MA |
| - Acide citrique qs pH = 7 | |
| - Eau déminéralisée | qsp 100,0 g |

COMPOSITION (B)

| | |
|---|---|
| - Métaperiodate de sodium | 3,5 g |
| - Alcool éthylique | 5,0 g |
| - Acide citrique qs pH = 4 | |
| - Eau déminéralisée | qsp 100,0 g |

La composition (A) est appliquée 20 minutes sur des cheveux gris à 90% de blancs. Après rinçage, on applique 15 minutes la composition (B). On rince alors à nouveau et on sèche. Les cheveux sont colorés en final dans une nuance blond foncé cendré.

EXEMPLE 20

COMPOSITION (A)

| | |
|---|---|
| - 7-amino indole | 1,0 g |
| - Alcool éthylique | 10,0 g |
| - Dérivé de gomme de guar vendu sous la dénomination de JAGUAR HP 60 par la Société MEYHALL | 1,0 g |
| - Alkyléther de glycoside vendu sous la dénomination de TRITON CG 110 par la Société ROHM & HAAS | 5,0 g MA |
| - Conservateurs qs | |
| - pH spontané = 6,7 | |
| - Eau déminéralisée | qsp 100,0 g |

COMPOSITION (B)

| | |
|---|---|
| - Métaperiodate de sodium | 5,0 g |
| - Acide chlorhydrique qs pH = 3 | |
| - Eau déminéralisée | qsp 100,0 g |

On applique 15 minutes la composition (A) sur des cheveux gris à 90% de blancs. On rince, puis on applique la composition (B) pendant 15 minutes. Après rinçage et séchage, on obtient des cheveux colorés dans une nuance blond foncé.

EXEMPLES 21 à 25

On procède à la teinture des cheveux en appliquant 60 g de la composition colorante ci-après. On laisse agir 10 minutes. On rince abondamment à l'eau, on essore les cheveux puis on applique 75 g de la composition oxydante ci-après qu'on laisse poser 10 minutes pour les exemples 21 à 23 et 20 minutes pour les exemples 24 à 25. Après rinçage et shampooing, on obtient la coloration indiquée en bas du tableau.

| en g | 21 | 22 | 23 | 24 | 25 |
|---|---|---|---|---|---|
| Composition colorante | | | | | |
| 6-aminoindole | 2 | | | | |
| 5-aminoindole | | 0,5 | | | |
| 6-ß-hydroxyéthylaminoindole | | | 2 | | |
| 4-aminoindole | | | | 1 | |
| 6-amino 2,3-diméthyl 5-hydroxy-indole, 2HBr | | | | | 0,5 |
| 5,6-dihydroxyindole | | | | | 0,5 |
| Alcool éthylique | 10 | 10 | 10 | 10 | |
| Hydroxyéthylcellulose vendue sous la dénomination NATROSOL 250 HHR par la Société HERCULES | 1 | 1 | 1 | 1 | |
| Alkyléther de glycoside vendu à 60% de MA sous la dénomination TRITON CG 110 par la Société SEPPIC | 5 (MA) | 5 (MA) | 5 (MA) | 5 (MA) | |
| Lauryléthersulfate de sodium à 28% de MA | | | | | 4,2 (MA) |
| Ether monobutylique de l'éthylène-glycol | | | | | 10 |
| Triéthanolamine qs pH | | | | | 5,2 |
| pH spontané | 6,5 | 6,5 | 6,5 | 6,9 | |
| Eau qsp | 100 | 100 | 100 | 100 | 100 |

| en g | 21 | 22 | 23 | 24 | 25 |
|---|---|---|---|---|---|
| Composition oxydante : | 1/3A + 2/3B | 1/3A + 2/3B | 1/3A + 2/3B | 1/3A + 2/3B | 2/3A + 1/3B |
| A) Nonylphénol oxyéthyléné à 4 moles d'oxyde d'éthylène | 26 | 26 | 26 | 26 | 26 |
| Nonylphénol oxyéthyléné à 9 moles d'oxyde d'éthylène | 24 | 24 | 24 | 24 | 24 |
| Ether monobutylique de l'éthylèneglycol | 13 | 13 | 13 | 13 | 13 |
| Propylèneglycol | 8 | 8 | 8 | 8 | 8 |
| Solution aqueuse d'ammoniaque à 20% | 19 | 19 | 19 | | 19 |
| Monoéthanolamine | | | | 8 | |
| Diéthanolamide d'acide oléique | 3,5 | 3,5 | 3,5 | 3,5 | 3,5 |
| Parfums, conservateurs, séquestrant | qs | qs | qs | qs | qs |
| Eau qsp | 100 | 100 | 100 | 100 | 100 |
| B) Eau oxygénée à 20 volumes | | | | | |
| Nuances obtenues : | blond foncé cuivré doré | acajou cuivré | blond doré cendré | blond beige doré | blond foncé doré mat |

EXEMPLE 26

COMPOSITION (A)

| | |
|---|---|
| - Chlorhydrate de 2,3-diméthyl 6-amino indole | 1,0 g |
| - Iodure de potassium | 1,0 g |
| - Hydroxyéthylcellulose vendue sous la dénomination NATROSOL 250 HHR par la Société AQUALON | 1,0 g |
| - Alkyléther de glycoside vendu à 60% de MA sous la dénomination TRITON CG 110 par la Société ROHM & HAAS | 5,0 g MA |
| - Lauryl éther sulfate de sodium | 0,2 g MA |
| - Triéthanolamine qs pH = 6,5 | |
| - Eau déminéralisée | qsp 100,0 g |

COMPOSITION (B)

Solution d'eau oxygénée à 12,5 volumes.

La composition (A) est appliquée pendant 15 minutes sur des cheveux gris à 90% de blancs. Après rinçage, on applique pendant 15 minutes la composition (B). On rince, on lave au shampooing et on sèche.

Les cheveux sont teints en blond clair cendré doré.

EXEMPLE 27

On procède comme dans l'exemple 26, en utilisant 1 g de chlorhydrate de 2,3,7-triméthyl 6-amino indole à la place du chlorhydrate de 2,3-diméthyl 6-amino indole.

La teinture s'effectue sur des cheveux gris permanentés. Ceux-ci sont colorés en blond nacré cuivré.

EXEMPLE 28

COMPOSITION (A)

| | |
|---|---|
| - Chlorhydrate de 2,3,5-triméthyl 6-amino indole | 0,5 g |
| - Iodure de potassium | 0,5 g |
| - Hydroxyéthylcellulose vendue sous la dénomination NATROSOL 250 HHR par la Société AQUALON | 1,0 g |
| - Alkyléther de glycoside vendu à 60% de MA sous la dénomination TRITON CG 110 par la Société ROHM & HAAS | 5,0 g MA |
| - Triéthanolamine qs pH = 6,5 | |
| - Eau déminéralisée | qsp 100,0 g |

COMPOSITION (B)

Solution d'eau oxygénée à 12,5 volumes.

La composition (A) est appliquée pendant 15 minutes sur des cheveux gris permanentés. Après rinçage, on applique pendant 15 minutes la composition (B). Après rinçage et shampooing, les cheveux sont teints en blond clair cendré doré.

EXEMPLE 29

COMPOSITION (A)

| | |
|---|---|
| - Sulfate de cuivre à 5 molécules d'eau | 1,0 g |
| - Lauryl éther sulfate de sodium à 2 moles d'oxyde d'éthylène, vendu sous la dénomination SACTIPON 8533 par la Société LEVER | 3,0 g MA |
| - Monoéthanolamine qs pH = 9,5 | |
| - Eau déminéralisée | qsp 100,0 g |

COMPOSITION (B)

| | |
|---|---|
| - Monochlorhydrate de 2,3,4,5-tétraméthyl 6-amino indole | 0,25 g |
| - Lauryl éther sulfate de sodium à 2 moles d'oxyde d'éthylène, vendu sous la dénomination SACTIPON 8533 par la Société LEVER | 4,2 g MA |
| - Monobutyléther d'éthylèneglycol | 10,0 g |
| - 2-amino 2-méthyl 1-propanol qs pH = 8,9 | |
| - Eau déminéralisée | qsp 100,0 g |

On applique la composition (A) pendant 10 minutes sur des cheveux gris à 90% de blancs. Après rinçage, ces cheveux sont traités pendant 15 minutes avec la composition (B). Après un nouveau rinçage et un séchage, les cheveux sont colorés dans une nuance blond très clair doré mat.

EXEMPLE 30

COMPOSITION (A)

| | |
|---|---|
| - Métapériodate de sodium | 5,0 g |
| - Acide chlorhydrique qs pH = 3,0 | |
| - Eau déminéralisée | qsp 100,0 g |

COMPOSITION (B)

| | |
|---|---|
| - Monochlorhydrate de 2,3,4,5-tétraméthyl 6-amino indole | 0,25 g |
| - Lauryl éther sulfate de sodium à 2 moles d'oxyde d'éthylène, vendu sous la dénomination SACTIPON 8533 par la Société LEVER | 4,2 g MA |
| - Monobutyléther d'éthylèneglycol | 10,0 g |
| - 2-amino 2-méthyl 1-propanol qs pH = 8,9 | |
| - Eau déminéralisée | qsp 100,0 g |

La composition (A) est appliquée pendant 10 minutes sur des cheveux gris à 90% de blancs. On rince et on traite ces mêmes cheveux pendant 15 minutes avec la composition (B). On rince à nouveau et on sèche. Les cheveux sont alors colorés dans une nuance blond cuivré nacré.

EXEMPLE 31

COMPOSITION (A)

| | |
|---|---|
| - Permanganate de potassium | 1,2 g |
| - Acide chlorhydrique qs pH = 5,0 | |
| - Eau déminéralisée | qsp 100,0 g |

COMPOSITION (B)

| | |
|---|---|
| - 2-méthyl 6-amino indole | 0,1 g |
| - Monochlorhydrate de N-méthyl 6-hydroxyéthylamino indole | 0,1 g |
| - Monochlorhydrate de 2,3-diméthyl 5-chloro 6-amino indole | 0,05 g |
| - Nonylphénol à 9 moles d'oxyde d'éthylène, vendu sous la dénomination SINNOPAL NP9 par la Société HENKEL | 3,0 g |
| - Polyéthylèneglycol 300 | 10,0 g |
| - Bromure de tétradécyl triméthyl ammonium | 0,1 g |
| - Monoéthyléther de l'éthylèneglycol | 4,0 g |
| - Triéthyanolamine qs pH = 6,6 | |
| - Eau déminéralisée | qsp 100,0 g |

Sur des cheveux gris à 90% de blancs, on applique pendant 10 minutes la composition (A) et on rince. On applique alors pendant 15 minutes la composition (B) et on rince à nouveau. Les cheveux présentent alors une coloration châtain clair cuivré doré.

EXEMPLE 32

COMPOSITION (A)

| | |
|---|---|
| - Sulfate de cuivre à 5 molécules d'eau | 1,0 g |
| - Lauryl éther sulfate de sodium à 2 moles d'oxyde d'éthylène, vendu sous la dénomination SACTIPON 8533 par la Société LEVER | 3,0 g MA |
| - Monoéthanolamine qs pH = 9,5 | |
| - Eau déminéralisée | qsp 100,0 g |

COMPOSITION (B)

| | |
|---|---|
| - Monochlorhydrate de 2,3-diméthyl 5-éthyl 6-amino indole | 0,2 g |
| - Tensio-actif amphotère dénommé Cocoamphocarboxyglycinate dans le dictionnaire CTFA, vendu sous la dénomination MIRANOL C2M par la Société MIRANOL | 3,0 g MA |
| - 1,2-propanediol | 12,0 g |
| - Soude qs pH = 7,8 | |
| - Eau déminéralisée | qsp 100,0 g |

Des cheveux gris à 90% de blancs sont imprégnés pendant 10 minutes avec la composition (A). Après rinçage, ils sont imprégnés pendant 15 minutes avec la composition (B). Après rinçage final et séchage, on

obtient des cheveux teints dans une nuance blond beige doré.

EXEMPLE 33

COMPOSITION (A)

| | |
|---|---|
| - Dibromhydrate de 2-méthyl 5-hydroxy 6-amino indole | 0,25 g |
| - Dibromhydrate de 2,3-diméthyl 5-hydroxy 6-amino indole | 0,15 g |
| - Tensio-actif amphotère dénommé Cocoamphocarboxyglycinate dans le dictionnaire CTFA, vendu sous la dénomination MIRANOL C2M par la Société MIRANOL | 4,0 g |
| - Monobutyléther de l'éthylèneglycol | 8,0 g |
| - Ethanol | 3,0 g |
| - N,N-diméthylaminoéthanol qs pH = 7,6 | |
| - Eau déminéralisée | qsp 100,0 g |

COMPOSITION (B)

| | |
|---|---|
| - Nitrite de sodium | 1,0 g |
| - Acide chlorhydrique qs pH = 3,0 | |
| - Eau déminéralisée | qsp 100,0 g |

On traite des cheveux gris à 90% de blancs pendant 15 minutes avec la composition (A). Après rinçage, on traite ces mêmes cheveux pendant 10 minutes avec la composition (B). On rince à nouveau et on sèche. Les cheveux sont colorés en final dans une nuance blond foncé doré cuivré.

EXEMPLE 34

COMPOSITION (A)

| | |
|---|---|
| - 2-méthyl 6-amino indole | 0,6 g |
| - Lauryl éther sulfate de sodium à 2 moles d'oxyde d'éthylène, vendu sous la dénomination SACTIPON 8533 par la Société LEVER | 2,8 g MA |
| - Nonylphénol à 9 moles d'oxyde d'éthylène, vendu sous la dénomination SINNOPAL NP9 par la Société HENKEL | 3,0 g |
| - Monométhyléther de propylèneglycol | 12,0 g |
| - Eau déminéralisée | qsp 100,0 g |
| - pH spontané = 8,5 | |

COMPOSITION (B)

| | |
|---|---|
| - Métapériodate de sodium | 5,0 g |
| - Acide chlorhydrique qs pH = 3,6 | |
| - Eau déminéralisée | qsp 100,0 g |

On applique pendant 15 minutes la composition (A) sur des cheveux gris à 90% de blancs. Après rinçage, les cheveux sont traités pendant 10 minutes avec la composition (B), puis rincés à nouveau. Après séchage, on obtient sur ces cheveux une coloration blond doré cuivré.

EXEMPLE 35

Les compositions (A) et (B) sont identiques à celles de l'exemple 34.

On inverse simplement l'ordre d'application des deux compositions en maintenant leurs temps de pause respectifs.

On obtient en final des cheveux teints dans une nuance blond foncé cuivré.

EXEMPLE 36

| | |
|---|---|
| - 2-méthyl 6-amino indole | 1,0 g |
| - Iodure de potassium | 1,0 g |
| - Monobutyléther de l'éthylèneglycol | 10,0 g |
| - Hydroxyéthylcellulose vendue sous la dénomination NATROSOL 250 HHR par la Société AQUALON | 1,0 g |
| - Alkyléther de glycoside vendu sous la dénomination TRITON CG 110 par la Société ROHM & HAAS | 5,0 g MA |
| - Conservateurs qs | |
| - Eau déminéralisée | qsp 100,0 g |
| - pH spontané = 7,4 | |

Cette composition est appliquée pendant 15 minutes sur des cheveux gris à 90% de blancs. On rince et on applique pendant 5 minutes un lait oxydant à pH3 titrant 12,5 volumes en eau oxygénée. Après un nouveau rinçage, on effectue un shampooing et on sèche. Les cheveux sont en final colorés dans une nuance blond cuivré doré puissante.

EXEMPLE 37

On remplace dans la composition de l'exemple 36 le 2-méthyl 6-amino indole par la même quantité de 6-N-($\beta$,$\gamma$-dihydroxypropyl)amino indole. Les conditions de teinture sont identiques.

Les cheveux sont colorés en une nuance blond foncé.

**Revendications**

**1.** Procédé de teinture des fibres kératiniques, en particulier des fibre kératiniques humaines, telles que les cheveux, caractérisé par le fait que l'on applique sur ces fibres une composition (A) contenant dans un milieu approprié pour la teinture, au moins un amino indole, répondant à la formule :

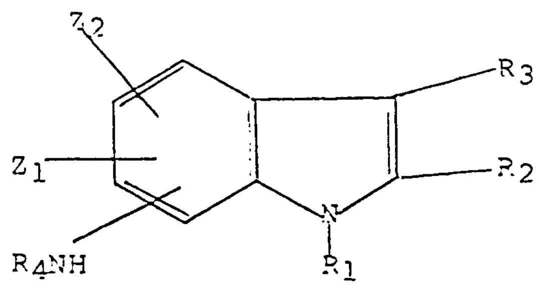

(I)

dans laquelle :

$R_1$ et $R_3$, indépendamment l'un de l'autre, représentent un atome d'hydrogène ou un groupement alkyle en $C_1$-$C_4$;

$R_2$ désigne hydrogène ou un groupement alkyle en $C_1$-$C_4$, COOR', R' étant un atome d'hydrogène ou un groupement alkyle en $C_1$-$C_4$;

$R_4$ représente un atome d'hydrogène ou un groupement alkyle en $C_1$-$C_4$, hydroxyalkyle en $C_1$-$C_4$, polyhydroxyalkyle en $C_2$-$C_4$;

$Z_1$ représente un atome d'hydrogène, d'halogène ou un groupement alkyle en $C_1$-$C_4$, ou OR;

R étant un atome d'hydrogène ou un groupement alkyle en $C_1$-$C_4$;
$Z_2$ représente un atome d'hydrogène ou un groupement alkyle en $C_1$-$C_4$;
et ses sels,
la couleur étant révélée à l'aide d'un système oxydant constitué par :
(i) des ions iodure et du peroxyde d'hydrogène, la composition (A) contenant dans ce cas en plus, soit (a) des ions iodure, soit (b) du peroxyde d'hydrogène et l'application de la composition (A) étant précédée ou suivie par l'application d'une composition (B) qui contient, dans un milieu approprié pour la teinture, soit :
(a) du peroxyde d'hydrogène à un pH compris entre 2 et 12 et de préférence entre 2 et 7, lorsque la composition (A) contient des ions iodure, soit
(b) des ions iodure à un pH compris entre 3 et 11, lorsque la composition (A) contient du peroxyde d'hydrogène;
(ii) des nitrites, l'application de la composition (A) étant suivie par l'application d'une composition aqueuse (B) présentant un pH acide, la composition (A) ou la composition (B) contenant au moins un nitrite;
(iii) des oxydants choisis parmi l'acide periodique et ses sels hydrosolubles, l'hypochlorite de sodium, le ferricyanure de potassium, l'oxyde d'argent, le réactif de Fenton, l'oxyde de plomb (IV), le sulfate de césium, le persulfate d'ammonium, ces oxydants étant présents dans la composition (A) ou appliqués simultanément ou séquentiellement de façon séparée, au moyen d'une composition (B) les contenant dans un milieu approprié pour la teinture;
(iv) des anions d'un métal choisi parmi les permanganates ou les bichromates, ces oxydants étant appliqués au moyen d'une composition (B) aqueuse à un pH de 2 à 10, avant l'application de la composition (A);
(v) des sels métalliques des groupes III à VIII du tableau périodique, ces sels métalliques étant appliqués dans une étape séparée au moyen d'une composition (B) contenant ces sels dans un milieu approprié pour la teinture;
(vi) des sels de terres rares, ces sels de terres rares étant appliqués au moyen d'une composition (B) les contenant dans un milieu approprié pour la teinture, la composition (B) étant appliquée préalablement ou postérieurement à l'application de la composition (A).

2. Procédé selon la revendication 1, caractérisé par le fait que les dérivés d'amino indole de formule (I) sont choisis parmi le 4-amino indole, le 5-amino indole, le 6-amino indole, le 7-amino indole, le 5-amino 6-méthoxy 2,3-diméthyl indole, le 6-amino 5-méthoxy 2,3-diméthyl indole, le 5-amino 6-hydroxy 2,3-diméthyl indole, le 5-hydroxy 6-amino 2,3-diméthyl indole, le 6-N-$\beta$-hydroxyéthylamino indole, le 6-N-$\beta$-hydroxyéthylamino 1-méthyl indole, le 6-méthylamino indole, le (5 ou 6)-amino N-méthyl indole, le 2-carboxy 6-amino indole, le 4-amino 2,3-diméthyl indole, le 6-amino 2,3-diméthyl indole, le 7-amino 2,3-diméthyl indole, le 6-amino 3-éthyl 2-méthyl indole, le 6-amino 3-méthyl indole, le 6-amino 2-méthyl indole, le 6-amino 2-éthoxycarbonyl indole, le 7-amino 3-éthyl 2-méthyl indole, le 6-N-($\beta$, $\gamma$-dihydroxypropyl)amino indole, le 2,3,4,5-tétraméthyl 6-amino indole, le 2,3-diméthyl 5-chloro 6-amino indole, le 2,3-diméthyl 5-éthyl 6-amino indole, le 2,3,4-triméthyl 6-amino indole, le 2-méthyl 5-hydroxy 6-amino indole, le 4-méthylamino indole, le 4-amino 1-méthyl indole, le 2,3-diméthyl 6-amino indole, le 2,3,7-triméthyl 6-amino indole, le 2,3,5-triméthyl 6-amino indole, et leurs sels.

3. Procédé de teinture des fibres kératiniques, en particulier des fibres kératiniques humaines telles que les cheveux, caractérisé par le fait que l'on applique sur ces fibres dans un premier temps, une composition tinctoriale contenant dans un support cosmétiquement acceptable aqueux et ayant un pH inférieur ou égal à 7, au moins un dérivé indolique répondant à la formule :

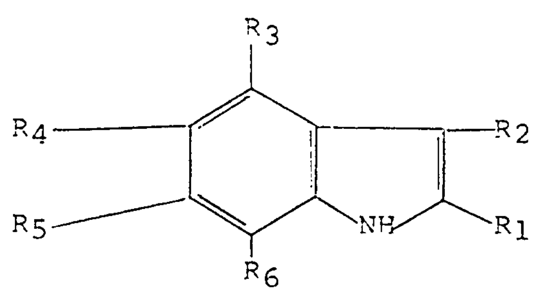

(II)

dans laquelle :

$R_1$, $R_2$, identiques ou différents, désignent H ou $CH_3$;

$R_3$ désigne H, $NH_2$, OH, $-OCH_3$;

$R_4$ désigne H, $NH_2$, OH, $-OC_2H_5$;

$R_5$ désigne H, $NH_2$, OH, $-NHCH_2CH_2OH$;

$R_6$ désigne H, OH;

deux au moins des radicaux $R_3$, $R_4$, $R_5$ et $R_6$ désignant hydrogène, et au moins un et un seul des groupements $R_3$, $R_4$ ou $R_5$ désigne $NH_2$ ou $-NHCH_2CH_2OH$ pour $R_5$; et lorsque $R_5$ désigne un groupement amino et $R_4$ désigne OH, $R_1$ et $R_2$ désignent le groupement méthyle, et les sels d'acides correspondants;

qu'après un temps de pose suivi d'un rinçage et d'un essorage, on applique, dans un second temps, une solution oxydante alcaline, cette application étant suivie d'un rinçage et d'un shampooing.

**4.** Procédé selon la revendication 3, caractérisé par le fait que les aminoindoles de formule (II) sont choisis parmi le 4-aminoindole, le 5-aminoindole, le 6-aminoindole, le 6-amino 2,3-diméthyl 5-hydroxyindole, le 6-$\beta$-hydroxyéthylaminoindole.

**5.** Procédé selon la revendication 1 ou 2, caractérisé par le fait que l'on applique sur les fibres une composition (A) contenant dans un milieu approprié pour la teinture, le colorant de formule (I), en association avec des ions iodure, l'application de la composition (A) étant précédée ou suivie par l'application d'une composition (B) qui contient dans un milieu approprié pour la teinture, du peroxyde d'hydrogène.

**6.** Procédé selon la revendication 1 ou 2, caractérisé par le fait que l'on applique une composition (A) contenant dans un milieu approprié pour la teinture le colorant de formule (I) en association avec du peroxyde d'hydrogène à un pH de 2 à 7, l'application de la composition (A) étant précédée ou suivie par l'application d'une composition (B) qui contient, dans un milieu approprié pour la teinture, des ions iodure.

**7.** Procédé selon la revendication 5 ou 6, caractérisé par le fait que les ions iodure sont choisis parmi les iodures de métaux alcalins, alcalino-terreux ou d'ammonium.

**8.** Procédé selon l'une quelconque des revendications 5 à 7, caractérisé par le fait que les ions iodure sont présents dans des proportions comprises entre 0,007 et 4% en poids exprimé en ions $I^-$ et de préférence entre 0,08 et 1,5% en poids par rapport au poids total de la composition (A) ou (B).

**9.** Procédé selon la revendication 1 ou 2, caractérisé par le fait que les nitrites sont choisis parmi les nitrites de métaux alcalins, alcalino-terreux ou d'ammonium, les dérivés organiques des nitrites et les vecteurs de nitrites, qui sont présents dans des proportions comprises entre 0,02 et 1 mole/litre.

**10.** Procédé selon la revendication 1 ou 2, caractérisé par le fait que les oxydants choisis parmi l'acide periodique et ses sels hydrosolubles, l'hypochlorite de sodium, le ferricyanure de potassium, l'oxyde d'argent, le réactif de Fenton, l'oxyde de plomb (IV), le sulfate de césium, le persulfate d'ammonium, sont présents dans des proportions comprises entre 0,004 et 0,07 mole, et en particulier entre 0,01 et 0,04 mole pour 100 g de composition.

**11.** Procédé selon la revendication 1 ou 2, caractérisé par le fait que l'anion de métal est constitué par le permanganate de potassium ou le bichromate de sodium utilisés à des molalités en anions supérieures à $10^{-3}$ mole/1000 g.

**12.** Procédé selon la revendication 1 ou 2, caractérisé par le fait que les sels métalliques du groupe III à VIII du tableau périodique sont choisis parmi les sels de manganèse, de cobalt, de fer, de cuivre et d'argent utilisés dans des proportions comprises entre 0,01 et 2% exprimées en ion métallique.

**13.** Procédé selon la revendication 12, caractérisé par le fait que l'application des compositions (A) et (B) est suivie par la mise en contact, après rinçage, avec une solution de peroxyde d'hydrogène pour éclaircir la couleur obtenue.

**14.** Procédé selon la revendication 1 ou 2, caractérisé par le fait que les sels de terres rares sont choisis parmi les sels de Cérium $Ce^{3+}$, $Ce^{4+}$, de Lanthane $La^{3+}$, d'Europium $Eu^{2+}$, $Eu^{3+}$, de Gadolinium $Gd^{3+}$, d'Ytterbium $Yb^{2+}$, $Yb^{3+}$, de Dysprosium $Dy^{3+}$.

**15.** Procédé selon l'une quelconque des revendications 1,2,5,6,13, caractérisé par le fait qu'on utilise des solutions de peroxyde d'hydrogène de 1 à 40 volumes, de préférence 2 à 10 volumes.

**16.** Procédé selon l'une quelconque des revendications 1 à 15, caractérisé par le fait que la composition (A) contient l'amino indole de formule (I) ou (II) dans des proportions comprises entre 0,01 et 5% en poids par rapport au poids total de la composition et de préférence entre 0,03 et 2,5% en poids.

**17.** Procédé selon l'une quelconque des revendications 1 à 16, caractérisé par le fait que les compositions (A) et (B) se présentent sous forme de lotions plus ou moins épaissies, de crèmes, de mousses, de gels, éventuellement conditionnées en aérosols.

**18.** Procédé selon l'une quelconque des revendications 1 à 17, caractérisé par le fait que le milieu approprié pour la teinture est un milieu aqueux, cosmétiquement acceptable lorsque les compositions sont destinées à la teinture des cheveux humains, ce milieu aqueux pouvant être constitué par de l'eau ou un mélange eau/solvant(s).

**19.** Procédé selon l'une quelconque des revendications 1 à 18, caractérisé par le fait que le milieu approprié pour la teinture est un milieu solvant anhydre.

**20.** Procédé selon la revendication 18 ou 19, caractérisé par le fait que les solvants sont choisis parmi l'alcool éthylique, l'alcool propylique, l'alcool isopropylique, l'alcool tertiobutylique, l'éthylène glycol, les éthers monométhylique, monoéthylique et monobutylique de l'éthylèneglycol, l'acétate du monoéthyléther de l'éthylèneglycol, le propylèneglycol, les monométhyléthers du propylèneglycol et du dipropylèneglycol, le lactate de méthyle.

**21.** Procédé selon l'une quelconque des revendications 1 à 18, caractérisé par le fait que le pH de la composition (A) est compris entre 2 et 7 et de préférence entre 3,5 et 7.

**22.** Procédé selon l'une quelconque des revendications 1 à 21, caractérisé par le fait que les compositions contiennent des amides gras, des agents tensio-actifs, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques et tout autre agent habituellement utilisé dans des compositions tinctoriales.

**23.** Procédé selon l'une quelconque des revendications 1 à 22, caractérisé par le fait que la composition tinctoriale renferme en plus d'autres colorants indoliques.

**24.** Procédé selon la revendication 23, caractérisé par le fait que les autres colorants indoliques sont choisis parmi le 5,6-dihydroxyindole, le 5,6-dihydroxy 2-carboxyindole, le 2-méthyl 5,6-dihydroxy indole et son sel d'addition acide correspondant.

**25.** Procédé selon l'une quelconque des revendications 3 à 4, caractérisé par le fait que la solution oxydante contient un oxydant choisi parmi le peroxyde d'hydrogène, le peroxyde d'urée, les persels choisis parmi les percarbonates, les perborates alcalins ou d'ammonium.

**26.** Procédé selon l'une quelconque des revendications 3, 4 et 25, caractérisé par le fait que les agents alcalins utilisés dans la solution oxydante sont choisis parmi l'ammoniaque et les alcanolamines.

**27.** Procédé selon l'une quelconque des revendications 3, 4 et 25, caractérisé par le fait que la proportion d'agent oxydant dans les compositions oxydantes que l'on applique sur les cheveux, est comprise entre 1 et 10% en poids par rapport au poids total de la composition alcaline oxydante et de préférence entre 1 et 5%.

**28.** Agent de coloration des fibres kératiniques, en particulier des fibres kératiniques humaines à plusieurs composants, caractérisé par le fait que l'un des composants est constitué par la composition (A) définie dans l'une quelconque des revendications 1 à 27 et que l'autre des composants est constitué par l'une quelconque des compositions (B) définie dans l'une quelconque des revendications 1 à 27.

**29.** Dispositif à plusieurs compartiments ou "kits" de teinture ou "nécessaire de teinture", caractérisé par le fait qu'il comprend un premier compartiment contenant la composition (A) comportant le dérivé d'amino indole de formule (I) dans un milieu approprié pour la teinture, tel que défini dans l'une quelconque des revendications 1 à 27, et un second compartiment contenant une composition (B) également définie dans l'une quelconque des revendications 1 à 27.

**30.** Composition destinée à être utilisée dans les colorations des fibres kératiniques, caractérisée par le fait qu'elle contient dans un milieu approprié pour la teinture, au moins un amino indole répondant à la formule (I) définie dans la revendication 1 ou 3, et au moins des ions iodure tels que définis dans la revendication 1 ou 7.

**31.** Composition destinée à être utilisée dans les colorations des fibres kératiniques, caractérisée par le fait qu'elle contient dans un milieu approprié pour la teinture, au moins un amino indole répondant à la formule (I) définie dans la revendication 1, et au moins un nitrite tel que défini dans la revendication 1 ou 9.

**32.** Composé nouveau répondant à la formule :

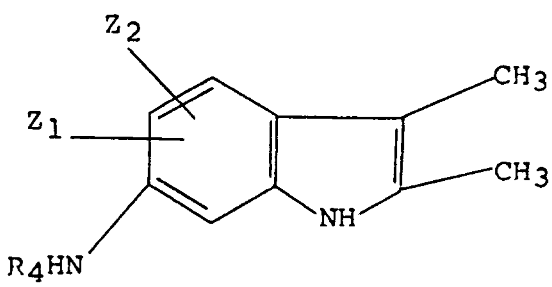

(IA)

dans laquelle :

$R_4$ désigne hydrogène, alkyle en $C_1$-$C_4$,hydroxyalkyle en $C_1$-$C_4$, polyhydroxyalkyle en $C_2$-$C_4$;

$Z_1$ désigne hydrogène, alkyle ou halogène

$Z_2$ désigne hydrogène, alkyle en $C_1$-$C_4$,sous réserve qu'au moins un des radicaux $Z_1$ ou $Z_2$ soit différent d'hydrogène à l'exclusion du 2,3,5-triméthyl 6-aminoindole.

**33.** Composé selon la revendication 32, caractérisé par le fait qu'il est choisi parmi le 2,3,7-triméthyl 6-amino indole, le 2,3,4,5-tétraméthyl 6-amino indole, le 2,3-diméthyl 5-éthyl 6-amino indole, le 2,3-diméthyl 5-chloro 6-amino indole, le 2,3,4-triméthyl 6-amino indole.

## Claims

**1.** Method for dyeing keratinous fibres, in particular human keratinous fibres, such as hair, characterized in that a composition (A) containing, in a medium appropriate for dyeing, at least one aminoindole corresponding to the formula:

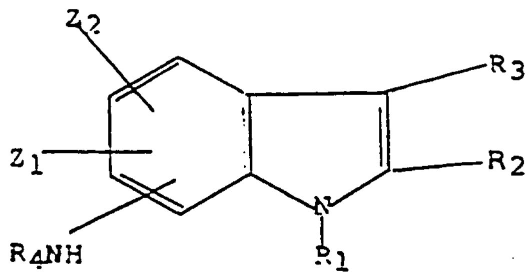

(I)

in which:

$R_1$ and $R_3$, independently of one another, represent a hydrogen atom or a $C_1$-$C_4$ alkyl group;

$R_2$ denotes hydrogen or a $C_1$-$C_4$ alkyl group or COOR', R' being a hydrogen atom or a $C_1$-$C_4$ alkyl group;

$R_4$ represents a hydrogen atom or a $C_1$-$C_4$ alkyl, $C_1$-$C_4$ hydroxyalkyl or $C_2$-$C_4$ polyhydroxyalkyl group;

$Z_1$ represents a hydrogen or halogen atom or a $C_1$-$C_4$ alkyl group or OR;

R being a hydrogen atom or a $C_1$-$C_4$ alkyl group; and

$Z_2$ represents a hydrogen atom or a $C_1$-$C_4$ alkyl group;

and its salts

is applied to said fibres, the colour being revealed with the aid of an oxidizing system consisting of:

(i) iodide ions and hydrogen peroxide, the composition (A) in this case also containing either (a) iodide ions or (b) hydrogen peroxide and the application of the composition (A) being preceded or followed by the application of a composition (B) which contains, in a medium appropriate for dyeing, either:

(a) hydrogen peroxide at a pH of between 2 and 12 and preferably between 2 and 7, when the composition (A) contains iodide ions, or

(b) iodide ions at a pH of between 3 and 11, when the composition (A) contains hydrogen peroxide;

(ii) nitrites, the application of the composition (A) being followed by the application of an aqueous composition (B) having an acid pH, the composition (A) or the composition (B) containing at least one nitrite;

(iii) oxidizing agents chosen from periodic acid and its water-soluble salts, sodium hypochlorite, potassium ferricyanide, silver oxide, Fenton's reagent, lead(IV) oxide, caesium sulphate and ammonium persulphate, these oxidizing agents being present in the composition (A) or being applied separately, at the same time or subsequently, by means of a composition (B) containing them in a medium appropriate for dyeing;

(iv) metal anions chosen from the permanganates or bichromates, these oxidizing agents being applied by means of an aqueous composition (B) having a pH of from 2 to 10, before the application of the composition (A);

(v) salts of metals of groups III to VIII of the periodic table, these metal salts being applied in a separate step by means of a composition (B) containing these salts in a medium appropriate for dyeing;

(vi) rare earth salts, these rare earth salts being applied by means of a composition (B) containing them in a medium appropriate for dyeing, the composition (B) being applied before or after the application of the composition (A).

**2.** Method according to Claim 1, characterized in that the aminoindole derivatives of formula (I) are chosen from 4-aminoindole, 5-aminoindole, 6-aminoindole, 7-aminoindole, 5-amino-6-methoxy-2,3-dimethylindole, 6-amino-5-methoxy-2,3-dimethylindole, 5-amino-6-hydroxy-2,3-dimethylindole, 5-hydroxy-6-amino-2,3-dimethylindole, 6-N-$\beta$-hydroxyethylaminoindole, 6-N-$\beta$-hydroxyethylamino-1-methylindole, 6-methylaminoindole, (5 or 6)-amino-N-methylindole, 2-carboxy-6-aminoindole, 4-amino-2,3-dimethylindole, 6-amino-2,3-dimethylindole, 7-amino-2,3-dimethylindole, 6-amino-3-ethyl-2-methylindole, 6-amino-3-methylindole, 6-amino-2-methylindole, 6-amino-2-ethoxycarbonylindole, 7-amino-3-ethyl-2-methylindole, 6-N-($\beta$,$\gamma$-dihydroxypropyl)aminoindole, 2,3,4,5-tetramethyl-6-aminoindole, 2,3-dimethyl-5-chloro-6-aminoindole, 2,3-dimethyl-5-ethyl-6-aminoindole, 2,3,4-trimethyl-6-aminoindole, 2-methyl-5-hydroxy-6-aminoindole, 4-methylaminoindole, 4-amino-1-methylindole, 2,3-dimethyl-6-aminoindole, 2,3,7-trimethyl-6-aminoindole, 2,3,5-trimethyl-6-aminoindole and their salts.

**3.** Method for dyeing keratinous fibres, in particular human keratinous fibres such as hair, characterized in that a tinctorial composition containing, in a cosmetically acceptable carrier having a pH of less than or equal to 7, at least one indole derivative corresponding to the formula:

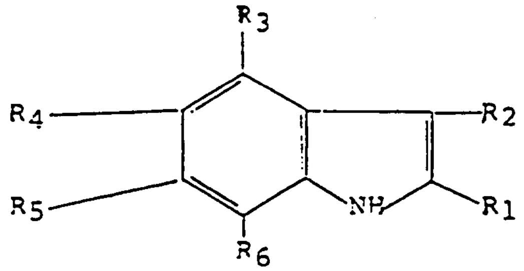

(II)

in which:

$R_1$ and $R_2$, which may be identical or different, denote H or $CH_3$;
$R_3$ denotes H, $NH_2$, OH or $-OCH_3$;
$R_4$ denotes H, $NH_2$, OH or $-OC_2H_5$;
$R_5$ denotes H, $NH_2$, OH or $-NHCH_2CH_2OH$; and
$R_6$ denotes H or OH;

at least two of the radicals $R_3$, $R_4$, $R_5$ and $R_6$ denoting hydrogen, and at least one and one only of the groups $R_3$, $R_4$ or $R_5$ denoting $NH_2$, or $-NHCH_2CH_2OH$ for $R_5$; and when $R_5$ denotes an amino group and $R_4$ denotes OH, $R_1$ and $R_2$ denote the methyl group, and the salts of corresponding acids, is initially applied to said fibres and in that after an exposure time followed by rinsing and drying, an alkaline oxidying solution is subsequently applied, this application being followed by rinsing and shampooing.

**4.** Method according to Claim 3, characterized in that the aminoindoles of formula (II) are chosen from 4-aminoindole, 5-aminoindole, 6-aminoindole, 6-amino-2,3-dimethyl-5-hydroxyindole and 6-$\beta$-hydroxyethylaminoindole.

**5.** Method according to Claim 1 or 2, characterized in that a composition (A) containing, in a medium appropriate for dyeing, the dye of formula (I) in combination with iodide ions is applied to the fibres, the application of the composition (A) being preceded or followed by the application of a composition (B) which contains hydrogen peroxide, in a medium appropriate for dyeing.

**6.** Method according to Claim 1 or 2, characterized in that a composition (A) containing, in a medium appropriate for dyeing, the dye of formula (I) in combination with hydrogen peroxide at a pH of from 2 to 7 is applied, the application of the composition (A) being preceded or followed by the application of a composition (B) which contains iodide ions, in a medium appropriate for dyeing.

**7.** Method according to Claim 5 or 6, characterized in that the iodide ions are chosen from alkali metal iodides, alkaline earth metal iodides or ammonium iodide.

**8.** Method according to any one of Claims 5 to 7, characterized in that the iodide ions are present in proportions of between 0.007 and 4% by weight, expressed as $I^-$ ions, and preferably between 0.08 and 1.5% by weight relative to the total weight of the composition (A) or (B).

**9.** Method according to Claim 1 or 2, characterized in that the nitrites are chosen from alkali metal or alkaline earth metal nitrites or ammonium nitrite, organic derivatives of nitrites and nitrite carriers, which are present in proportions of between 0.02 and 1 mole/litre.

**10.** Method according to Claim 1 or 2, characterized in that the oxidying agents chosen from periodic acid and its water-soluble salt, sodium hypochlorite, potassium ferricyanide, silver oxide, Fenton's reagent, lead(IV) oxide, caesium sulphate and ammonium persulphate are present in proportions of between

0.004 and 0.07 mole and in particular between 0.01 and 0.04 mole per 100 g of composition.

**11.** Method according to Claim 1 or 2, characterized in that the metal anion is potassium permanganate or sodium bichromate used in anion molar amounts of more than $10^{-3}$ moles/1,000 g.

**12.** Method according to Claim 1 or 2, characterized in that the salts of metals of group III to VIII of the period table are chosen from manganese, cobalt, iron, copper and silver salts used in proportions of between 0.01 and 2%, expressed as metal ion.

**13.** Method according to Claim 12, characterized in that the application of the compositions (A) and (B) is followed by bringing into contact, after rinsing, with a hydrogen peroxide solution to lighten the colour obtained.

**14.** Method according to Claim 1 or 2, characterized in that the rare earth salts are chosen from the cerium $Ce^{3+}$ and $Ce^{4+}$, lanthanum $La^{3+}$, europium $Eu^{2+}$ and $Eu^{3+}$, gadolinium $Gd^{3+}$, ytterbium $Yb^{2+}$ and $Yb^{3+}$ and dysprosium $Dy^{3+}$ salts.

**15.** Method according to any one of Claims 1, 2, 5, 6 and 13, characterized in that 1 to 40 volume, preferably 2 to 10 volume hydrogen peroxide solutions are used.

**16.** Method according to any one of Claims 1 to 15, characterized in that the composition (A) contains the aminoindole of formula (I) or (II) in proportions of between 0.01 and 5% by weight relative to the total weight of the composition, and preferably between 0.03 and 2.5% by weight.

**17.** Method according to any one of Claims 1 to 16, characterized in that the compositions (A) and (B) are in the form of lotions, thickened to a greater or lesser extent, creams, foams or gels, if appropriate packaged in aerosols.

**18.** Method according to any one of Claims 1 to 17, characterized in that the medium appropriate for dyeing is an aqueous medium, which is cosmetically acceptable when the compositions are intended for dyeing human hair, it being possible for said aqueous medium to consist of water or a water/solvent(s) mixture.

**19.** Method according to any one of Claims 1 to 18, characterized in that the medium appropriate for dyeing is an anhydrous solvent medium.

**20.** Method according to Claim 18 or 19, characterized in that the solvents are chosen from ethyl alcohol, propyl alcohol, isopropyl alcohol, tertiary butyl alcohol, ethylene glycol, ethylene glycol monomethyl, monoethyl and monobutyl ethers, ethylene glycol monoethyl ether-acetate, propylene glycol, propylene glycol and dipropylene glycol monomethyl ethers, and methyl lactate.

**21.** Method according to any one of Claims 1 to 18, characterized in that the pH of the composition (A) is between 2 and 7 and preferably between 3.5 and 7.

**22.** Method according to any one of Claims 1 to 21, characterized in that the compositions contain fatty amides, surfactants, thickeners, perfumes, sequestering agents, film-forming agents, treatment agents, dispersing agents, conditioning agents, preservatives, opacifying agents, agents for swelling keratinous fibres and any other agent customarily used in tinctorial compositions.

**23.** Process according to any one of Claims 1 to 22, characterized in that the tinctorial composition also contains other indole dyes.

**24.** Method according to Claim 23, characterized in that the other indole dyes are chosen from 5,6-dihydroxyindole, 5,6-dihydroxy-2-carboxyindole, 2-methyl-5,6-dihydroxyindole and its corresponding acid addition salt.

**25.** Method according to any one of Claims 3 to 4, characterized in that the oxidizing solution contains an oxidizing agent chosen from hydrogen peroxide, urea peroxide and per salts chosen from alkali metal

or ammonium percarbonates or perborates.

**26.** Method according to any one of Claims 3, 4 and 25, characterized in that the alkaline agents used in the oxidizing solution are chosen from ammonia and alkanolamines.

**27.** Method according to any one of Claims 3, 4 and 25, characterized in that the proportion of oxidizing agent in the oxidizing compositions which are applied to the hair is between 1 and 10% by weight relative to the total weight of the alkaline oxidizing composition, and preferably between 1 and 5%.

**28.** Agent for dyeing keratinous fibres, in particular human keratinous fibres, containing several components, characterized in that one of the components consists of the composition (A) defined in any one of Claims 1 to 27 and in that the other component consists of any one of the compositions (B) defined in any one of Claims 1 to 27.

**29.** Multi-compartment device or dyeing "kits" or "dyeing set", characterized in that it comprises a first compartment containing the composition (A) containing the aminoindole derivative of formula (I) in a medium appropriate for dyeing, as defined in any one of Claims 1 to 27, and a second compartment containing a composition (B) also defined in any one of Claims 1 to 27.

**30.** Composition intended to be used in dyeing keratinous fibres, characterized in that it contains, in a medium appropriate for dyeing, at least one aminoindole corresponding to the formula (I) defined in Claim 1 or 3, and at least iodide ions as defined in Claim 1 or 7.

**31.** Composition intended to be used in dyeing keratinous fibres, characterized in that it contains, in a medium appropriate for dyeing, at least one aminoindole corresponding to the formula (I) defined in Claim 1, and at least one nitrite as defined in Claim 1 or 9.

**32.** New compound, corresponding to the formula:

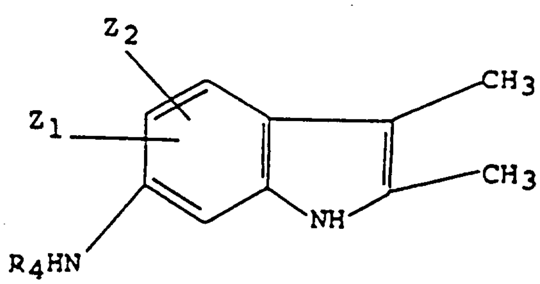

(IA)

in which:

$R_4$ denotes hydrogen, $C_1$-$C_4$ alkyl, $C_1$-$C_4$ hydroxyalkyl or $C_2$-$C_4$ polyhydroxyalkyl;
$Z_1$ denotes hydrogen, alkyl or halogen; and
$Z_2$ denotes hydrogen or $C_1$-$C_4$ alkyl;

with the proviso that at least one of the radicals $Z_1$ or $Z_2$ differs from hydrogen, with the exception of 2,3,5-trimethyl-6-aminoindole.

**33.** Compound according to Claim 32, characterized in that it is chosen from 2,3,7-trimethyl-6-aminoindole, 2,3,4,5-tetramethyl-6-aminoindole, 2,3-dimethyl-5-ethyl-6-aminoindole, 2,3-dimethyl-5-chloro-6-aminoindole and 2,3,4-trimethyl-6-aminoindole.

**Patentansprüche**

**1.** Verfahren zur Färbung keratinischer Fasern, insbesondere menschlicher keratinischer Fasern, wie der Haare,
dadurch **gekennzeichnet**, daß
man auf diese Fasern eine Zusammensetzung (A) aufträgt, enthaltend, in einem zur Färbung geeigneten Milieu, mindestens ein Aminoindol der Formel:

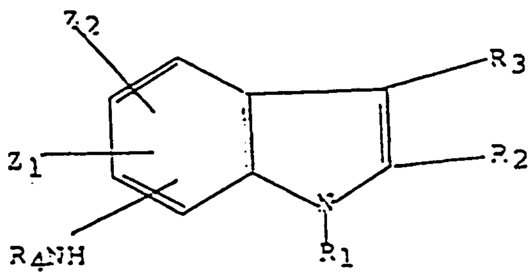

(I)

worin gilt:
$R_1$ und $R_3$ stellen, unabhängig voneinander, ein Wasserstoffatom oder eine $C_{1-4}$-Alkylgruppe dar;
$R_2$ bedeutet Wasserstoff oder eine $C_{1-4}$-Alkyl- oder COOR'-Gruppe, worin R' ein Wasserstoffatom oder eine $C_{1-4}$-Alkylgruppe ist;
$R_4$ stellt ein Wasserstoffatom oder eine $C_{1-4}$-Alkyl-, $C_{1-4}$-Hydroxyalkyl- oder $C_{2-4}$-Polyhydroxyalkylgruppe dar;
$Z_1$ stellt ein Wasserstoff- oder Halogenatom, eine $C_{1-4}$-Alkyl- oder OR-Gruppe dar, worin R ein Wasserstofftom oder eine $C_{1-4}$-Alkylgruppe ist;
$Z_2$ stellt ein Wasserstoffatom oder eine $C_{1-4}$-Alkylgruppe dar;
sowie seine Salze,
wobei die Farbe mit einem oxidierenden System entwickelt wird, das zusammengesetzt ist aus:

(i) Jodidionen und Wasserstoffperoxid, wobei die Zusammensetzung (A) in diesem Fall zusätzlich entweder (a) Jodidionen oder (b) Wasserstoffperoxid enthält und die Aufbringung der Zusammensetzung (A) vor oder nach der Aufbringung einer Zusammensetzung (B) erfolgt, die, in einem zur Färbung geeigneten Milieu, entweder:

(a) Wasserstoffperoxid bei einem pH von 2 bis 12 und vorzugsweise von 2 bis 7, wenn die Zusammensetzung (A) Jodidionen enthält, oder

(b) Jodidionen bei einem pH von 3 bis 11 enthält, wenn die Zusammensetzung (A) Wasserstoffperoxid enthält;

(ii) Nitriten, wobei nach der Aufbringung der Zusammensetzung (A) die Aufbringung einer wässrigen Zusammensetzung (B) erfolgt, die einen sauren pH aufweist, und wobei die Zusammensetzung (A) oder die Zusammensetzung (B) mindestens ein Nitrit enthalten;

(iii) Oxidationsmitteln, ausgewählt aus Perjodsäure und ihren wasserlöslichen Salzen, Natriumhypochlorit, Kaliumferricyanid, Silberoxid, Fenton-Reagens, Blei(IV)oxid, Cäsiumsulfat, Ammoniumpersulfat, wobei diese Oxidationsmittel in der Zusammensetzung (A) vorliegen oder gleichzeitig oder aufeinanderfolgend in getrennter Weise mit einer Zusammensetzung (B) aufgetragen werden, die jene in einem zur Färbung geiegneten Milieu enthält;

(iv) metallhaltigen Anionen, ausgewählt aus Permanganaten oder Bichromaten, wobei diese Oxidationsmittel mit einer wässrigen Zusammensetzung (B) bei einem pH von 2 bis 10 vor der Aufbringung der Zusammensetzung (A) aufgetragen werden;

(v) Metallsalzen der Gruppen III bis VIII des Periodensystems, wobei diese Metallsalze in einer getrennten Stufe mit einer Zusammensetzung (B) aufgetragen werden, die diese Salze in einem zur Färbung geeigneten Milieu enthält;

(vi) Salzen von seltenen Erden, wobei diese Salze der seltenen Erden mit einer Zusammensetzung (B) aufgetragen werden, die jene in einem zur Färbung geeigneten Milieu enthält, wobei die Zusammensetzung (B) vor oder nach der Aufbringung der Zusammensetzung (A) aufgetragen wird.

**2.** Verfahren gemäß Anspruch 1,
dadurch **gekennzeichnet**, daß
die Aminoindolderivate der Formel (I) aus 4-Aminoindol, 5-Aminoindol, 6-Aminoindol, 7-Aminoindol, 5-Amino-6-methoxy-2,3-dimethylindol, 6-Amino-5-methoxy-2,3-dimethylindol, 5-Amino-6-hydroxy-2,3-dimethylindol, 5-Hydroxy-6-amino-2,3-dimethylindol, 6-N-$\beta$-Hydroxyethylaminoindol, 6-N-$\beta$-Hydroxyethylamino-1-methylindol, 6-Methylaminoindol, (5 oder 6)-Amino-N-methylindol, 2-Carboxy-6-aminoindol, 4-Amino-2,3-dimethylindol, 6-Amino-2,3-dimethylindol, 7-Amino-2,3-dimethylindol, 6-Amino-3-ethyl-2-methylindol, 6-Amino-3-methylindol, 6-Amino-2-methylindol, 6-Amino-2-ethoxycarbonylindol, 7-Amino-3-ethyl-2-methylindol, 6-N-($\beta$,$\gamma$-Dihydroxypropyl)aminoindol, 2,3,4,5-Tetramethyl-6-aminoindol, 2,3-Dimethyl-5-chlor-6-aminoindol, 2,3-Dimethyl-5-ethyl-6-aminoindol, 2,3,4-Trimethyl-6-aminoindol, 2-Methyl-5-hydroxy-6-aminoindol, 4-Methylaminoindol, 4-Amino-1-methylindol, 2,3-Dimethyl-6-aminoindol, 2,3,7-Trimethyl-6-aminoindol, 2,3,5-Trimethyl-6-aminoindol sowie aus deren Salzen ausgewählt sind.

**3.** Verfahren zur Färbung keratinischer Fasern, insbesondere menschlicher keratinischer Fasern wie der Haare,
dadurch **gekennzeichnet**, daß
man auf diese Fasern in einer ersten Stufe eine Färbezusammensetzung, enthaltend in einem kosmetisch verträglichen, wässrigen Trägermilieu, das einen pH unterhalb oder gleich 7 aufweist, mindestens ein Indolderivat der Formel:

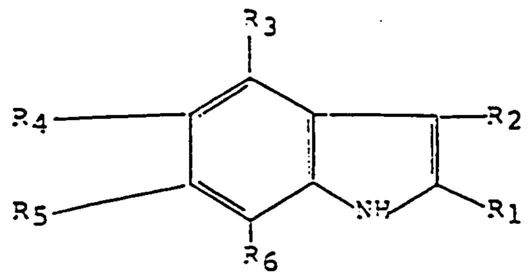

(II)

worin gilt:
$R_1$, $R_2$, gleich oder verschieden, bezeichnen H oder $CH_3$;
$R_3$ bezeichnet H, $NH_2$, OH, $-OCH_3$;
$R_4$ bezeichnet H, $NH_2$, OH, $-OC_2H_5$;
$R_5$ bezeichnet H, $NH_2$, OH, $-NHCH_2CH_2OH$;
$R_6$ bezeichnet H, OH,
wobei mindestens zwei der Reste $R_3$, $R_4$, $R_5$ und $R_6$ Wasserstoff darstellen,
und wobei mindestens eine und eine einzige der Gruppen $R_3$, $R_4$ oder $R_5$ $NH_2$oder $-NHCH_2CH_2OH$ für $R_5$ bedeuten; und wenn $R_5$ eine Aminogruppe und $R_4$ eine OH-Gruppe bedeuten, stellen $R_1$ und $R_2$ $CH_3$ dar,
sowie die entsprechenden Salze von Säuren,
und nach einer Verweilzeit und anschließender Spülung und Lufttrocknung in einer zweiten Stufe eine oxidierende alkalische Lösung aufträgt, worauf gespült und schamponiert wird.

**4.** Verfahren gemäß Anspruch 3,
dadurch **gekennzeichnet**, daß
die Aminoindole der Formel (II) aus 4-Aminoindol, 5-Aminoindol, 6-Aminoindol, 6-Amino-2,3-dimethyl-5-hydroxyindol, 6-$\beta$-Hydroxyethylaminoindol ausgewählt sind.

**5.** Verfahren gemäß Anspruch 1 oder 2,
dadurch **gekennzeichnet**, daß
man auf die Fasern eine Zusammensetzung (A) aufträgt, die, in einem zur Färbung geeigneten Milieu, den Farbstoff der Formel (I) zusammen mit Jodidionen enthält, wobei vor oder nach der Aufbringung der Zusammensetzung (A) die Aufbringung einer Zusammensetzung (B) erfolgt, die, in einem zur Färbung geeigneten Milieu, Wasserstoffperoxid enthält.

**6.** Verfahren gemäß Anspruch 1 oder 2,
dadurch **gekennzeichnet**, daß
man eine Zusammensetzung (A) aufträgt, die, in einem zur Färbung geeigneten Milieu, den Farbstoff der Formel (I) zusammen mit Wasserstoffperoxid bei einem pH von 2 bis 7 enthält, wobei vor oder nach der Aufbringung der Zusammensetzung (A) die Aufbringung einer Zusammensetzung (B) erfolgt, die, in einem zur Färbung geeigneten Milieu,Jodidionen enthält.

**7.** Verfahren gemäß Anspruch 5 oder 6,
dadurch **gekennzeichnet**, daß
die Jodidionen aus Alkali-, Erdalkalimetall-, Ammoniumjodiden ausgewählt sind.

**8.** Verfahren gemäß jedem der Ansprüche 5 bis 7,
dadurch **gekennzeichnet**, daß
die Jodidionen in Mengenverhältnissen von 0,007 bis 4 Gew.%, vorzugsweise 0,08 bis 1,5 Gew.%, ausgedrückt in $J^-$-Ionen, bezogen auf das Gesamtgewicht der Zusammensetzung (A) oder (B), vorhanden sind.

**9.** Verfahren gemäß Anspruch 1 oder 2,
dadurch **gekennzeichnet**, daß
die Nitrite aus Alkali-, Erdalkalimetall-, oder Ammoniumnitriten, organischen Nitritderivaten und Vektoren von Nitriten ausgewählt sind, die in Mengenverhältnissen von 0,02 bis 1 Mol/l vorliegen.

**10.** Verfahren gemäß Anspruch 1 oder 2,
dadurch **gekennzeichnet**, daß
die Oxidationsmittel, die aus Perjodsäure und ihren wasserlöslichen Salzen, Natriumhypochlorit, Kaliumferricyanid, Silberoxid, Fenton-Reagens, Blei(IV)oxid, Cäsiumsulfat, Ammoniumpersulfat ausgewählt sind, in Mengenverhältnissen von 0,004 bis 7 Mol, insbesondere von 0,01 bis 0,4 Mol, auf 100 g Zusammensetzung vorhanden sind.

**11.** Verfahren gemäß Anspruch 1 oder 2,
dadurch **gekennzeichnet**, daß
das Metallanion aus Kaliumpermanganat oder Natriumbichromat zusammengesetzt ist, welche in Molalitäten an Anionen oberhalb $10^{-3}$ Mol/1000 g verwendet werden.

**12.** Verfahren gemäß Anspruch 1 oder 2,
dadurch **gekennzeichnet**, daß
die Metallsalze der Gruppe III bis VIII des Periodensystems aus Salzen von Mangan, Kobalt, Eisen, Kupfer und Silber ausgewählt sind, welche in Mengenverhältnissen von 0,01 bis 2%, ausgedrückt in Metallion, verwendet werden.

**13.** Verfahren gemäß Anspruch 12,
dadurch **gekennzeichnet**, daß
nach der Aufbringung der Zusammensetzung (A) und (B), nach Spülung, die Aufbringung einer wässrigen Lösung von Wasserstoffperoxid erfolgt, um den erhaltenen Farbton aufzuhellen.

**14.** Verfahren gemäß Anspruch 1 oder 2,
dadurch **gekennzeichnet**, daß
die Salze der seltenen Erden aus Salzen von Cer $Ce^{3+}$, $Ce^{4+}$, Lanthan $La^{3+}$, Europium $Eu^{2+}$, $Eu^{3+}$, Gadolinium $Gd^{3+}$, Ytterbium $Yb^{2+}$, $Y^{3+}$, Dysprosium $Dy^{3+}$ ausgewählt sind.

**15.** Verfahren gemäß jedem der Ansprüche 1, 2, 5, 6, 13,
dadurch **gekennzeichnet**, daß
man Lösungen von Wasserstoffperoxid von 1 bis 40 Volumina, vorzugsweise 2 bis 10 Volumina, einsetzt.

**16.** Verfahren gemäß jedem der Ansprüche 1 bis 15,
dadurch **gekennzeichnet**, daß
die Zusammensetzung (A) das Aminoindol der Formel (I) oder (II) in Mengenverhältnissen von 0,01 bis 5 Gew.%, vorzugsweise von 0,03 bis 2,5 Gew.%, bezogen auf das Gesamtgewicht der Zusammensetzung, enthalt.

**17.** Verfahren gemäß jedem der Ansprüche 1 bis 16,
dadurch **gekennzeichnet**, daß
die Zusammensetzungen (A) und (B) in Form von mehr oder weniger verdickten Lotionen, Creme-Produkten, Schäumen, Gelen, welche gegebenenfalls als Aerosole zubereitet sind, vorliegen.

**18.** Verfahren gemäß jedem der Ansprüche 1 bis 17,
dadurch **gekennzeichnet**, daß
das zur Färbung geeignete Milieu ein kosmetisch verträgliches, wässriges Milieu ist, wenn die

Zusammensetzungen zur Färbung menschlicher Haare bestimmt sind, wobei dieses wässrige Milieu aus Wasser oder einer Mischung aus Wasser/Lösungsmittel(n) zusammengesetzt ist.

**19.** Verfahren gemäß jedem der Ansprüche 1 bis 18,
dadurch **gekennzeichnet**, daß
das zur Färbung geeignete Milieu ein wasserfreies Lösungsmittelmilieu ist.

**20.** Verfahren gemäß Anspruch 18 oder 19,
dadurch **gekennzeichnet**, daß
die Lösungsmittel aus Ethyl-, Propyl-, Isopropyl-, t-Butylalkohol, Ethylenglycol, den Monomethyl-, Monoethyl- und Monobutylethern von Ethylenglycol, Monoethyletherethylenglycolacetat, Propylenglycol, den Monomethylethern von Propylenglycol und Dipropylenglycol, Methyllactat ausgewählt sind.

**21.** Verfahren gemäß jedem der Ansprüche 1 bis 18,
dadurch **gekennzeichnet**, daß
der pH der Zusammensetzung (A) 2 bis 7, vorzugsweise 3,5 bis 7,beträgt.

**22.** Verfahren gemäß jedem der Ansprüche 1 bis 21,
dadurch **gekennzeichnet**, daß
die Zusammensetzungen Fettamide, oberfächenative Mittel, Verdickungsmittel, Parfüm-Produkte, Sequestriermittel, filmbildende Mittel, Behandlungsmittel, Dispergiermittel, Konditioniermittel, Konservierungsmittel, opak machende Mittel, Auflockerungsmittel für keratinische Fasern und jedes weitere gewöhnlich in Färbezusammenetzungen eingesetzte Mittel enthalten.

**23.** Verfahren gemäß jedem der Ansprüche 1 bis 22,
dadurch **gekennzeichnet**, daß
die Färbezusammensetzung zusätzlich weitere indolische Farbstoffe umfaßt.

**24.** Verfahren gemäß Anspruch 23,
dadurch **gekennzeichnet**, daß
die weiteren indolischen Farbstoffe aus 5,6-Dihydroxyindol, 5,6-Dihydroxy-2-carboxyindol, 2-Methyl-5,6-dihydroxyindol und seinem entsprechenden Säureadditionssalz ausgewählt sind.

**25.** Verfahren gemäß jedem der Ansprüche 3 bis 4,
dadurch **gekennzeichnet**, daß
die oxidierende Lösung ein Oxidationsmittel enthält, ausgewählt aus Wasserstoffperoxid, Harnstoffperoxid, Persalzen, ausgewählt aus Alkali- oder Ammoniumpercarbonaten, -perboraten.

**26.** Verfahren gemäß jedem der Ansprüche 3, 4 und 25,
dadurch **gekennzeichnet**, daß
die in der oxidierenden Lösung eingesetzten alkalisch machenden MIttel aus Ammoniak und Alkanolaminen ausgewählt sind.

**27.** Verfahren gemäß jedem der Ansprüche 3, 4 und 25,
dadurch **gekennzeichnet**, daß
das Mengenverhältnis an oxidierendem Mittel in den oxidierenden Zusammensetzungen, die man auf die Haare aufbringt, 1 bis 10 Gew.%, vorzugsweise 1 bis 5 Gew.%, bezogen auf das Gesamtgewicht der alkalischen oxidierenden Zusammensetzung beträgt.

**28.** Mittel aus mehreren Bestandteilen zur Färbung keratinischer Fasern, insbesondere menschlicher keratinischer Fasern,
dadurch **gekennzeichnet**, daß
der eine der Bestandteile aus der in jedem der Ansprüche 1 bis 27 definierten Zusammensetzung (A) und der andere der Bestandteile aus einer jeden der in jedem der Ansprüche 1 bis 27 definierten Zusammensetzungen (B) zusammengesetzt sind.

**29.** Vorrichtung aus mehreren Teilstücken oder "Kits zur Färbung" oder "Necessaire zur Färbung",
dadurch **gekennzeichnet**, daß

sie ein erstes Teilstück, enthaltend die Zusammensetzung (A), enthaltend in einem zur Färbung geeigneten Milieu das in einem jeden der Ansprüche 1 bis 27 definierte Aminoindolderivat der Formel (I), und ein zweites Teilstück umfassen, das eine ebenfalls in einem jeden der Ansprüche 1 bis 27 definierte Zusammensetzung (B) enthält.

**30.** Zusammensetzung zur Verwendung in Färbungen keratinischer Fasern,
dadurch **gekennzeichnet**, daß
sie, in einem zur Färbung geeigneten Milieu, mindestens ein Aminoindol der in Anspruch 1 oder 3 definierten Formel (I) und mindestens Jodidionen enthält, wie in Anspruch 1 oder 7 definiert.

**31.** Zusammensetzung zur Verwendung in Färbungen keratinischer Fasern,
dadurch **gekennzeichnet**, daß
sie , in einem zur Färbung geeigneten Milieu, mindestens ein Aminoindol der in Anspruch 1 definierten Formel (I) sowie mindestens ein in Anspruch 1 oder 9 definiertes Nitrit enthält.

**32.** Neue Verbindung der Formel:

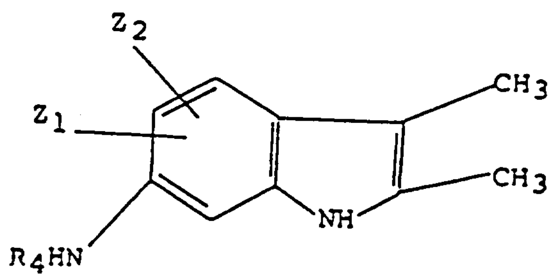

(IA)

worin gilt:
$R_4$ bedeutet Wasserstoff, eine $C_{1-4}$-Alkyl-, $C_{1-4}$-Hydroxyalkyl- oder $C_{2-4}$Polyhydroxyalkylgruppe;
$Z_1$ bedeutet Wasserstoff, eine Alkylgruppe oder ein Halogenatom;
$Z_2$ bedeutet Wasserstoff oder eine $C_{1-4}$-Alkylgruppe,
mit der Maßgabe, daß mindestens einer der Reste $Z_1$ oder $Z_2$ verschieden von Wasserstoff ist, ausgenommen 2,3,5-Trimethyl-6-aminoindol.

**33.** Verbindung gemäß Anspruch 32,
dadurch **gekennzeichnet**, daß
sie aus 2,3,7-Trimethyl-6-aminoindol, 2,3,4,5-Tetramethyl-6-aminoindol, 2,3-Dimethyl-5-ethyl-6-aminoindol, 2,3-Dimethyl-5-chlor-6-aminoindol, 2,3,4-Trimethyl-6-aminoindol ausgewählt ist.